(19) Europäisches Patentamt
European Patent Office
Office européen des brevets

(11) Veröffentlichungsnummer : **0 577 558 A2**

# (12) EUROPÄISCHE PATENTANMELDUNG

(21) Anmeldenummer : **93810446.0**

(22) Anmeldetag : **22.06.93**

(51) Int. Cl.[5] : **C07D 239/54,** C07D 239/46, C07D 239/48, C07D 473/00, C07C 275/50, C07C 215/44, C07F 9/6561, C07F 9/6512, C08G 79/04, A61K 31/52, A61K 31/505

(30) Priorität : **01.07.92 CH 2075/92**

(43) Veröffentlichungstag der Anmeldung : **05.01.94 Patentblatt 94/01**

(84) Benannte Vertragsstaaten : **BE CH DE ES FR GB IT LI NL SE**

(71) Anmelder : **CIBA-GEIGY AG**
**Klybeckstrasse 141**
**CH-4002 Basel (CH)**

(72) Erfinder : **Altmann, Karl-Heinz, Dr.**
**Vogesenstrasse 46**
**CH-4056 Basel (CH)**
Erfinder : **Imwinkelried, René, Dr.**
**Klosterweg 1**
**CH-3902 Brig-Glis (CH)**
Erfinder : **Eschenmoser, Albert, Prof. Dr.**
**Bergstrasse 9**
**CH-8700 Küsnacht (CH)**

(54) **Carbocyclische Nukleoside mit bicyclischen Ringen, Oligonukleotide daraus, Verfahren zu deren Herstellung, deren Verwendung und Zwischenproduckte.**

(57) Verbindungen der Formeln I und Ia und ihre Racemate

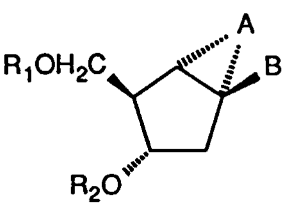
(I),
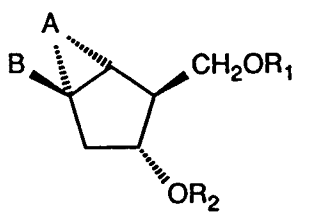
(Ia),

worin A für $-CH_2-$ oder $-CH_2CH_2-$ steht, $R_1$ für Wasserstoff oder eine Schutzgruppe steht, $R_2$ für Wasserstoff oder eine Schutzgruppe oder einen eine phosphorhaltige Nukleotid-Brückengruppe bildenden Rest steht und B einen Purin- oder Pyrimidinrest oder ein Analoges davon bedeutet, können als antivirale Wirkstoffe oder zur Herstellung biologisch aktiver Oligonukleotide verwendet werden.

EP 0 577 558 A2

Die Erfindung betrifft Nukleosidanaloge mit einem Bicyclo-[3.1.0]-hexan- oder Bicyclo-[3.2.0]-heptangerüst, ein Verfahren zu deren Herstellung durch an sich bekannte Aufbaureaktionen von Nukleinbasen aus der Thymin-, Adenin-, Purin- oder Cytosinreihe, 1-Amino-3-hydroxy-4-hydroxymethyl-bicyclo-[3.1.0]-hexan sowie 1-Amino-3-hydroxy-4-hydroxymethyl-bicyclo-[3.2.0]-heptan und dessen geschützte Derivate als Zwischenprodukte, Oligonukleotide mit diesen Nukleosiden und die Verwendung der Nukleoside zur Herstellung von Oligonukleotiden mit gleichen oder verschiedenen Nukleosideinheiten im Molekül.

Nukleoside und Oligonukleotide haben als antivirale Wirkstoffe bzw. wegen ihrer Fahigkeit zur Wechselwirkung mit Nukleinsäuren ("Antisense"-Oligonukleotide) und der damit verbundenen biologischen Aktivität breites Interesse gefunden, siehe zum Beispiel E. Uhlmann et al., Chemical Reviews 90:543-584 (1990). Zur Bereitstellung von Nukleosiden mit neuen Eigenschaften oder zur Verbesserung der Wechselwirkung von Antisense-Oligonukleotiden mit natürlichen Nukleinsäuren sowie ihrer Stabilität gegenüber Nukleasen sind die Zuckerreste von Nukleosiden (bzw. der Nukleotideinheiten in Oligo-nukleotiden) oder die internukleotidische Phosphatbindung in Oligonukleotiden in unterschiedlichster Weise modifiziert worden, siehe zum Beispiel V. E. Marquez et al., Medicinal Research Reviews 6:140 (1986), C. Hélène et al., Biochimica et Biophysica Acta 1049:99-125 (1990), U. Englisch et al., Angewandte Chemie Heft 6, 629-646 (1991), M.D. Matteucci et al. in Annual Reports in Medicinal Chemistry, Academic Press Inc., 287-296 (1991) und WO 91/06556. Bicyclische und carbocyclische Nukleoside mit 3'-Hydroxy- und 4'-Hydroxymethylgruppen sind für diesen Zweck noch nicht bekannt geworden.

Ein Gegenstand der Erfindung sind enantiomere Verbindungen der Formeln I und Ia und ihre Racemate

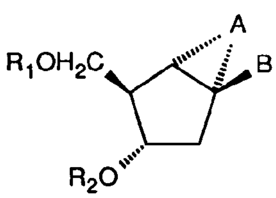

(I),

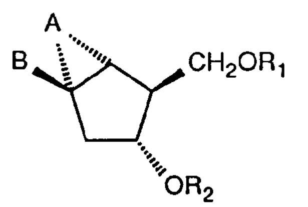

(Ia),

worin A für $-CH_2-$ oder $-CH_2CH_2-$ steht, $R_1$ für Wasserstoff oder eine Schutzgruppe steht, $R_2$ für Wasserstoff oder eine Schutzgruppe oder einen eine phosphorhaltige Nukleotid-Brückengruppe bildenden Rest steht und B einen Purin- oder Pyrimidinrest oder ein Analoges davon bedeutet.

Bevorzugt sind Verbindungen, in denen $R_1$ und $R_2$ je für Wasserstoff stehen.

In einer besonders bevorzugten Ausführungsform stellt A $-CH_2-$ dar.

In einer anderen bevorzugten Ausführungsform handelt es sich um die Enantiomeren der Formel I.

Schutzgruppen und Verfahren zur Derivatisierung der Hydroxylgruppen mit solchen Schutzgruppen sind in der Zucker- und Nukleotidchemie allgemein bekannt und zum Beispiel von B. T. Greene, Protective Groups in Organic Synthesis, Wiley Interscience, New York (1991) beschrieben. Beispiele für solche Schutzgruppen sind: lineares oder verzweigtes $C_1$-$C_8$-, besonders $C_1$-$C_4$-Alkyl, zum Beispiel Methyl, Ethyl, n- und i-Propyl, n-, i- und t-Butyl; $C_7$-$C_{18}$-Aralkyl, zum Beispiel Benzyl, Methylbenzyl, Dimethylbenzyl, Methoxybenzyl, Dimethoxybenzyl, Brombenzyl; Diphenylmethyl, Di(methylphenyl)methyl, Di(dimethylphenyl)methyl, Di(methoxyphenyl)methyl, Di(dimethoxyphenyl)methyl, Trityl, Tri(methylphenyl)methyl, Tri(dimethylphenyl)methyl, Methoxyphenyl(diphenyl)methyl, Di(methoxyphenyl)phenylmethyl, Tri(methoxyphenyl)methyl, Tri(dimethoxyphenyl)methyl; Triphenylsilyl, Alkyldiphenylsilyl, Dialkylphenylsilyl und Trialkylsilyl mit 1 bis 20, bevorzugt 1 bis 12 und besonders bevorzugt 1 bis 8 C-Atomen in den Alkylgruppen, zum Beispiel Trimethylsilyl, Triethylsilyl, Tri-n-propylsilyl, i-Propyldimethylsilyl, t-Butyl-dimethylsilyl, t-Butyl-diphenylsilyl, n-Octyl-dimethylsilyl, (1,1,2,2-Tetramethylethyl)-dimethylsilyl; $-(C_1$-$C_8$-Alkyl$)_2$Si-O-Si$(C_1$-$C_8$-Alkyl$)_2-$, worin Alkyl zum Beispiel Methyl, Ethyl, n- oder i-Propyl, n-, i- oder t-Butyl bedeutet; $C_2$-$C_{12}$-, besonders $C_2$-$C_8$-Acyl, wie zum Beispiel Acetyl, Propanoyl, Butanoyl, Pentanoyl, Hexanoyl, Benzoyl, Methylbenzoyl, Methoxybenzoyl, Chlorbenzoyl und Brombenzoyl; $R_3$-$SO_2$-, worin $R_3$ $C_1$-$C_{12}$-Alkyl, besonders $C_1$-$C_6$-Alkyl bedeutet, $C_5$- oder $C_6$-Cycloalkyl, Phenyl, Benzyl, $C_1$-$C_{12}$- und besonders $C_1$-$C_4$-Alkylphenyl, oder $C_1$-$C_{12}$- und besonders $C_1$-$C_4$-Alkylbenzyl, oder Halogenphenyl oder Halogenbenzyl bedeutet, zum Beispiel Methyl-, Ethyl-, Propyl-, Butyl-, Phenyl-, Benzyl-, p-Brom-, p-Methoxy- oder p-Methylphenylsulfonyl; unsubstituiertes oder mit F, Cl, Br, $C_1$-$C_4$-Alkoxy, Tri-($C_1$-$C_4$-Alkyl)silyl oder $C_1$-$C_4$-Alkylsulfonyl substituiertes $C_1$-$C_{12}$-, bevorzugt $C_1$-$C_8$-Alkoxycarbonyl, zum Beispiel Methoxy-, Ethoxy-, n- oder i-Propoxy- oder n-, i- oder t-Butoxycarbonyl, 2-Trimethylsilylethoxycarbonyl, 2-Methylsulfonylethoxycarbonyl, oder unsubstituiertes oder wie für Alkoxycarbonyl substituiertes Phenyloxycarbonyl oder Benzyloxycarbonyl, zum Beispiel Methyl- oder Methoxy- oder Chlorphenyloxycarbonyl oder -benzyloxycarbonyl, sowie 9-Fluorenylmethyloxycarbonyl. Sofern $R_1$ und/oder $R_2$ Alkyl bedeuten, kann es mit F, Cl, Br, $C_1$-$C_4$-Alkoxy, Phenyloxy, Chlorphenyloxy, Methoxyphenyloxy, Benzyloxy, Methoxybenzyloxy oder Chlorphenyloxy substi-

tuiert sein. Bei $R_1$ und $R_2$ in Formel I kann es sich um gleiche oder verschiedene Schutzgruppen handeln, wobei oft gleiche Schutzgruppen bevorzugt sind.

In einer bevorzugten Ausführungsform sind die Verbindungen der Formel I solche, worin $R_1$ und $R_2$ als Schutzgruppen unabhängig voneinander lineares oder verzweigtes $C_1$-$C_4$-Alkyl, $C_7$-$C_{18}$-Aralkyl, Trialkylsilyl mit 1 bis 12 C-Atomen in den Alkylgruppen, -($C_1$-$C_4$-Alkyl)$_2$Si-O-Si($C_1$-$C_4$-Alkyl)$_2$, $C_2$-$C_8$-Acyl, $R_3$-$SO_2$-, worin $R_3$ $C_1$-$C_6$-Alkyl, Phenyl, Benzyl, $C_1$-$C_4$-Alkylphenyl, $C_1$-$C_4$-Alkylbenzyl, Halogenphenyl oder Halogenbenzyl bedeutet, oder $C_1$-$C_8$-Alkoxycarbonyl, Phenoxycarbonyl oder Benzyloxycarbonyl oder 9-Fluorenylmethyloxycarbonyl darstellen.

In einer besonders bevorzugten Ausführungsform stellen $R_1$ und $R_2$ als Schutzgruppen Methyl, Ethyl, n- oder i-Propyl, n-, i- oder t-Butyl; Benzyl, Methylbenzyl, Dimethylbenzyl, Methoxybenzyl, Dimethoxybenzyl, Brombenzyl; Diphenylmethyl, Di(methylphenyl)methyl, Di(dimethylphenyl)methyl, Di(methoxyphenyl)methyl, Di(methoxyphenyl)(phenyl)methyl, Trityl, Tri(methylphenyl)methyl, Tri(dimethylphenyl)methyl, Tri(methoxyphenyl)methyl, Tri(dimethoxyphenyl)methyl; Trimethylsilyl, Triethylsilyl, Tri-n-propylsilyl, i-Propyl-dimethylsilyl, t-Butyl-dimethylsilyl, t-Butyl-diphenylsilyl, n-Octyl-dimethylsilyl, (1,1,2,2-Tetramethylethyl)-dimethylsilyl, -$(CH_3)_2$Si-O-Si$(CH_3)_2$-, -(i-$C_3H_7$)$_2$Si-O-Si(i-$C_3H_7$)$_2$-; Acetyl, Propanoyl, Butanoyl, Pentanoyl, Hexanoyl, Benzoyl, Methylbenzoyl, Methoxybenzoyl, Chlorbenzoyl und Brombenzoyl; Methyl-, Ethyl-, Propyl-, Butyl-, Phenyl-, Benzyl-, p-Brom-, p-Methoxy- und p-Methylphenylsulfonyl; Methoxy-, Ethoxy-, n- oder i-Propoxy- oder n-, i- oder t-Butoxycarbonyl, oder Phenyloxycarbonyl, Benzyloxycarbonyl, Methyl- oder Methoxy- oder Chlorphenyloxycarbonyl oder -benzyloxycarbonyl oder 9-Fluorenylmethyloxycarbonyl dar.

$R_2$ kann als phosphorhaltiger, eine Nukleotid-Brückengruppe bildender Rest der Formel

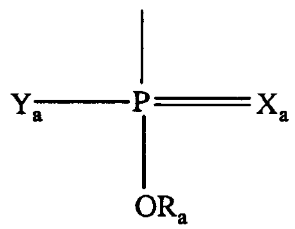

entsprechen, worin $Y_a$ Wasserstoff, $C_1$-$C_{12}$-Alkyl, $C_6$-$C_{12}$-Aryl, $C_7$-$C_{20}$-Aralkyl, $C_7$-$C_{20}$-Alkaryl, -$OR_b$, -$SR_b$, -$NH_2$, Primäramino, Sekundäramino, $O^{\ominus}M^{\oplus}$ oder $S^{\ominus}M^{\oplus}$ darstellt; $X_a$ Sauerstoff oder Schwefel bedeutet; $R_a$ Wasserstoff, $M^{\oplus}$, $C_1$-$C_{12}$-Alkyl, $C_2$-$C_{12}$-Alkenyl oder $C_6$-$C_{12}$-Aryl bedeutet, oder die Gruppe $R_a$O- für N-Heteroaryl-N-yl mit 5 Ringgliedern und 1 bis 3 N-Atomen steht; $R_b$ Wasserstoff, $C_1$-$C_{12}$-Alkyl oder $C_6$-$C_{12}$-Aryl bedeutet; und $M^{\oplus}$ für $Na^{\oplus}$, $K^{\oplus}$, $Li^{\oplus}$, $NH_4^{\oplus}$ steht oder Primär-, Sekundär-, Tertiär- oder Quartenärammonium darstellt; wobei Alkyl, Aryl, Aralkyl und Alkaryl in $Y_a$, $R_a$ und $R_b$ unsubstituiert oder mit Alkoxy, Alkylthio, Halogen, -CN, -$NO_2$, Phenyl, Nitrophenyl oder Halogenphenyl substituiert sind.

$Y_a$ enthält als Primäramino bevorzugt 1 bis 12 und besonders bevorzugt 1 bis 6 C-Atome, und als Sekundäramino bevorzugt 2 bis 12 und besonders bevorzugt 2 bis 6 C-Atome.

Bei dem Primäramino und Sekundäramino kann es sich zum Beispiel um Reste der Formel $R_cR_dN$ handeln, worin $R_c$ für H steht oder unabhängig die Bedeutung von $R_d$ hat, und $R_c$ $C_1$-$C_{20}$-, bevorzugt $C_1$-$C_{12}$- und besonders bevorzugt $C_1$-$C_6$-Alkyl, -Aminoalkyl, Hydroxyalkyl; Carboxyalkyl oder Carbalkoxyalkyl, wobei die Carbalkoxygruppe 2 bis 8 C-Atome und die Alkylgruppe 1 bis 6, bevorzugt 1 bis 4 C-Atome enthält; $C_2$-$C_{20}$-, bevorzugt $C_2$-$C_{12}$- und besonders bevorzugt $C_2$-$C_6$-Alkenyl; Phenyl, Mono- oder Di-($C_1$-$C_4$-Alkyl- oder -Alkoxy)phenyl, Benzyl, Mono- oder Di-($C_1$-$C_4$-Alkyl- oder -Alkoxy)benzyl; oder 1,2-, 1,3- oder 1,4-Imidazolyl-$C_1$-$C_6$-Alkyl darstellt, oder $R_c$ und $R_c$ zusammen Tetra- oder Pentamethylen, 3-Oxa-1,5-pentylen, -$CH_2$-$NR_e$-$CH_2CH_2$- oder -$CH_2CH_2$-$NR_{19}$-$CH_2CH_2$- darstellen, worin $R_e$ für H oder $C_1$-$C_4$-Alkyl steht. Die Aminogruppe im Aminoalkyl kann mit ein oder zwei $C_1$-$C_4$-Alkyl oder -Hydroxyalkylgruppen substituiert sein. Die Hydroxylgruppe im Hydroxyalkyl kann mit $C_1$-$C_4$-Alkyl verethert sein.

Unter Primär-, Sekundär-, Tertiär- und Quartärammonium ist für $Y_a$ im Zusammenhang mit der Definition von $M^{\oplus}$ ein Ion der Formel $R_fR_gR_hR_iN^{\oplus}$ zu verstehen, bei dem $R_f$ $C_1$-$C_{20}$-, bevorzugt $C_1$-$C_{12}$- und besonders bevorzugt $C_1$-$C_6$-Alkyl, -Aminoalkyl, -Hydroxyalkyl; Carboxyalkyl oder Carbalkoxyalkyl, wobei die Carbalkoxygruppe 2 bis 8 C-Atome und die Alkylgruppe 1 bis 6, bevorzugt 1 bis 4 C-Atome enthält; $C_2$-$C_{20}$-, bevorzugt $C_2$-$C_{12}$- und besonders bevorzugt $C_2$-$C_6$-Alkenyl; Phenyl, Mono- oder Di-($C_1$-$C_4$-Alkyl- oder -Alkoxy)phenyl, Benzyl, Mono- oder Di-($C_1$-$C_4$-Alkyl- oder -Alkoxy)benzyl; oder 1,2-, 1,3- oder 1,4-Imidazolyl-$C_1$-$C_6$-Alkyl darstellt, und $R_g$, $R_h$ und $R_i$ unabhängig voneinander Wasserstoff sind oder die Bedeutung von $R_f$ haben, oder $R_f$ und Rg zusammen Tetra- oder Pentamethylen, 3-Oxa-1,5-pentylen, -$CH_2$-$NR_e$-$CH_2CH_2$- oder -$CH_2CH_2$-$NR_e$-$CH_2CH_2$- darstellen, worin $R_e$ für H oder $C_1$-$C_4$-Alkyl steht, und $R_h$ und $R_i$ unabhängig vonein-

ander die Bedeutung von $R_f$ haben. Die Aminogruppe im Aminoalkyl kann mit ein oder zwei $C_1$-$C_4$-Alkyl oder -Hydroxyalkylgruppen substituiert sein. Die Hydroxylgruppe im Hydroxyalkyl kann mit $C_1$-$C_4$-Alkyl verethert sein.

Beispiele für Carboxyalkyl sind Carboxymethyl, Carboxyethyl, Carboxypropyl und Carboxybutyl, und Beispiele für Carbalkoxyalkyl sind diese mit Methyl oder Ethyl veresterten Carboxyalkylgruppen. Beispiele für Alkenyl sind Allyl, But-1-en-3-yl oder -4-yl, Pent-3- oder 4-en-1-yl oder -2-yl, Hex-3- oder -4- oder -5-en-1-yl oder -2-yl. Beispiele für Alkyl- und Alkoxyphenyl beziehungsweise -benzyl sind Methylphenyl, Dimethylphenyl, Ethylphenyl, Diethylphenyl, Methylbenzyl, Dimethylbenzyl, Ethylbenzyl, Diethylbenzyl, Methoxyphenyl, Dimethoxyphenyl, Ethoxyphenyl, Diethoxyphenyl, Methoxybenzyl, Dimethoxybenzyl, Ethoxybenzyl, Diethoxybenzyl. Beispiele für Imidazolylalkyl, in dem die Alkylgruppe bevorzugt 2 bis 4 C-Atome enthält, sind 1,2-, 1,3- oder 1,4-Imidazolylethyl oder -n-propyl oder -n-butyl. $R_{19}$ stellt bevorzugt H, Methyl oder Ethyl dar.

Bevorzugte Beispiele für Primäramino und Sekundäramino sind Methyl-, Ethyl-, Dimethyl-, Diethyl-, Di-i-Propyl, Mono- oder Di-(1-hydroxy-eth-2-yl)-, Phenyl- und Benzylamino, Acetylamino und Benzoylamino sowie Piperidinyl, Piperazinyl und Morpholinyl.

Bevorzugte Beispiele für Primär- und Sekundärammonium sind Methyl-, Ethyl-, Dimethyl-, Diethyl-, Di-i-Propyl-, Mono- oder Di-(1-hydroxy-eth-2-yl)-, Phenyl- und Benzylammonium.

Beispiele für $Y_a$, $R_a$ und $R_b$ als Alkyl sind Methyl, Ethyl und die Isomeren von Propyl, Butyl, Pentyl, Hexyl, Heptyl und Octyl; Beispiele für $Y_a$, $R_a$ und $R_b$ als Aryl sind Phenyl und Naphthyl; Beispiele für $R_a$ als Alkenyl sind Allyl und ($C_1$-$C_4$-Alkyl)CH=CH-$CH_2$-; Beispiele für $Y_a$ als Aralkyl sind Phenyl-$C_nH_{2n}$- mit n gleich einer Zahl von 1 bis 6, besonders Benzyl; Beispiele für $Y_a$ als Alkaryl sind Mono-, Di- und Tri($C_1$-$C_4$-alkyl)phenyl. Bevorzugte Substituenten sind Chlor, Brom, Methoxy, -$NO_2$, -CN, 2,4-Dichlorphenyl und 4-Nitrophenyl. Beispiele für $R_b$ sind 2,2,2-Trichlorethyl, 4-Chlorphenyl, 2-Chlorphenyl und 2,4-Dichlorphenyl; und Beispiele für $R_b$O- als N-Heteroaryl sind Pyrrol-N-yl, Triazol-N-yl und Benztriazol-N-yl.

In einer besonders bevorzugten Ausführungsform bedeutet $R_b$ β-Cyanoethyl, stellt $R_a$ Di(i-propylamino) dar und steht $X_a$ für O.

Wenn B einen Purinrest oder ein Analoges davon darstellt, so kann es sich um Reste der Formel II, IIa, IIb, IIc, IId oder IIe handeln,

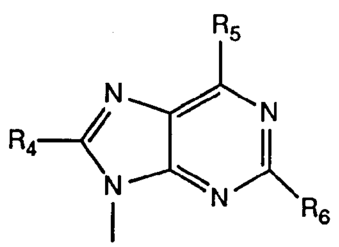

(II),

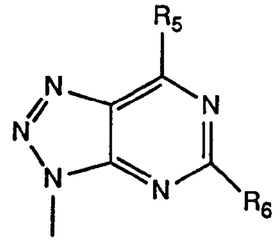

(IIa),

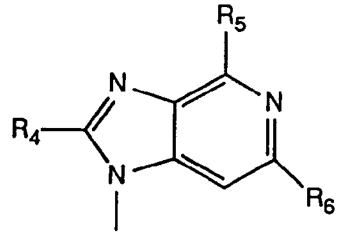

(IIb),

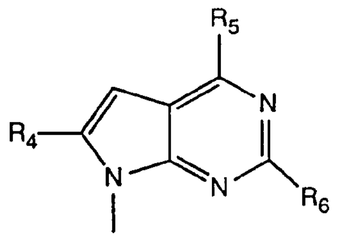

(IIc),

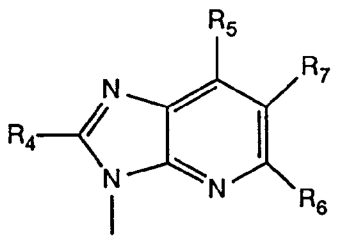

(IId),

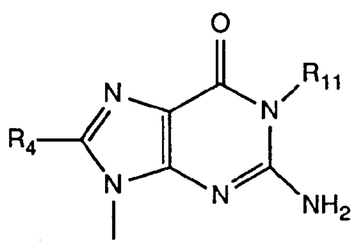

(IIe),

worin $R_4$ für H, Cl, Br, OH oder $C_1$-$C_{12}$-Alkoxy steht, und $R_5$, $R_6$ und $R_7$ unabhängig voneinander H, OH, SH, $NH_2$, $NHNH_2$, NHOH, NHOAlkyl mit 1 bis 12 C-Atomen, -N=CH-N($C_1$-$C_{12}$-Alkyl)$_2$, F, Cl, Br, Alkyl oder Hydroxyalkyl oder Aminoalkyl oder Alkoxy oder Alkylthio mit 1 bis 12 C-Atomen, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten, und $R_{11}$ H oder $C_1$-$C_4$-Alkyl darstellt.

Geeignete Schutzgruppen sind zuvor erwähnt worden. Bevorzugte Schutzgruppen sind $C_1$-$C_8$-Acylgruppen, wie zum Beispiel Acetyl, Propionyl, Butyroyl und Benzoyl. $R_{11}$ steht bevorzugt für H oder Methyl.

Das Primäramino enthält bevorzugt 1 bis 12 und besonders bevorzugt 1 bis 6 C-Atome, und das Sekundäramino bevorzugt 2 bis 12 und besonders bevorzugt 2 bis 6 C-Atome.

Einige Beispiele für Alkyl, Alkoxy, Alkylthio, Hydroxyalkyl und Aminoalkyl, die bevorzugt 1 bis 6 C-Atome enthalten, sind Methyl, Ethyl und die Isomeren von Propyl, Butyl, Pentyl, Hexyl, Heptyl, Octyl, Nonyl, Decyl, Undecyl und Dodecyl, sowie entsprechende Alkoxy-, Alkylthio-, Hydroxyalkyl- und Aminoalkylreste. Die Alkyl-, Alkoxy-, Alkylthio-, Hydroxyalkyl- und Aminoalkylreste enthalten besonders bevorzugt 1 bis 4 C-Atome. Bevorzugte Alkyl-, Alkoxy-, Alkylthio-, Hydroxyalkyl- und Aminoalkylreste sind Methyl, Ethyl, n- und i-Propyl, n-, i- und t-Butyl, Methoxy, Ethoxy, Methylthio und Ethylthio, Aminomethyl, Aminoethyl, Hydroxymethyl und Hydroxyethyl.

Bei dem Primäramino und Sekundäramino kann es sich zum Beispiel um Reste der Formel $R_8R_9N$ handeln, worin $R_8$ für H steht oder unabhängig die Bedeutung von $R_9$ hat, und $R_9$ $C_1$-$C_{20}$-, bevorzugt $C_1$-$C_{12}$- und besonders bevorzugt $C_1$-$C_6$-Alkyl, -Aminoalkyl, -Hydroxyalkyl; Carboxyalkyl oder Carbalkoxyalkyl, wobei die Carbalkoxygruppe 2 bis 8 C-Atome und die Alkylgruppe 1 bis 6, bevorzugt 1 bis 4 C-Atome enthält; $C_2$-$C_{20}$-, bevorzugt $C_2$-$C_{12}$- und besonders bevorzugt $C_2$-$C_6$-Alkenyl; Phenyl, Mono- oder Di($C_1$-$C_4$-Alkyl- oder -Alkoxy)phenyl, Benzyl, Mono- oder Di-($C_1$-$C_4$-Alkyl- oder -Alkoxy)benzyl; oder 1,2-, 1,3- oder 1,4-Imidazolyl-$C_1$-$C_6$-Alkyl darstellt, oder $R_8$ und $R_9$ zusammen Tetra- oder Pentamethylen, 3-Oxa-1,5-pentylen, -$CH_2$-$NR_{10}$-$CH_2CH_2$- oder -$CH_2CH_2$-$NR_{10}$-$CH_2CH_2$- darstellen, worin $R_{10}$ für H oder $C_1$-$C_4$-Alkyl steht. Die Aminogruppe im Aminoalkyl kann mit ein oder zwei $C_1$-$C_4$-Alkyl oder -Hydroxyalkylgruppen substituiert sein. Die Hydroxylgruppe im Hydroxyalkyl kann mit $C_1$-$C_4$-Alkyl verethert sein.

Beispiele für Alkyl sind zuvor angegeben worden. Beispiele für Aminoalkyl sind Aminomethyl, Aminoethyl, 1-Aminoprop-2-yl oder-3-yl, 1-Amino-but-2-yl oder -3-yl oder -4-yl, N-Methyl- oder N,N-Dimethyl- oder N-Ethyl- oder N,N-Diethyl- oder N-2-Hydroxyethyl- oder N,N-Di-2-hydroxyethylaminomethyl oder -aminoethyl oder

-aminopropyl oder -aminobutyl. Beispiele für Hydroxyalkyl sind Hydroxymethyl, 1-Hydroxy-eth-2-yl, 1-Hydroxy-prop-2-oder-3-yl, 1-Hydroxy-but-2-yl, -3-yl oder -4-yl. Beispiele für Carboxyalkyl sind Carboxymethyl, Carboxyethyl, Carboxypropyl und Carboxybutyl, und Beispiele für Carbalkoxyalkyl sind diese mit Methyl oder Ethyl veresterten Carboxyalkylgruppen. Beispiele für Alkenyl sind Allyl, But-1-en-3-yl oder -4-yl, Pent-3- oder 4-en-1-yl oder -2-yl, Hex-3- oder -4- oder -5-en-1-yl oder -2-yl. Beispiele für Alkyl- und Alkoxyphenyl beziehungsweise -benzyl sind Methylphenyl, Dimethylphenyl, Ethylphenyl, Diethylphenyl, Methylbenzyl, Dimethylbenzyl, Ethylbenzyl, Diethylbenzyl, Methoxyphenyl, Dimethoxyphenyl, Ethoxyphenyl, Diethoxyphenyl, Methoxybenzyl, Dimethoxybenzyl, Ethoxybenzyl, Diethoxybenzyl. Beispiele für Imidazolylalkyl, in dem die Alkylgruppe bevorzugt 2 bis 4 C-Atome enthält, sind 1,2-, 1,3- oder 1,4-Imidazolylethyl oder -n-propyl oder -n-butyl. $R_{10}$ stellt bevorzugt H, Methyl oder Ethyl dar.

Bevorzugte Beispiele für Primäramino und Sekundäramino sind Methyl-, Ethyl-, Dimethyl-, Diethyl-, Allyl-, Mono- oder Di-(1-hydroxy-eth-2-yl)-, Phenyl- und Benzylamino, Acetylamino, Isobutyrylamino und Benzoylamino.

In einer bevorzugten Ausführungsform stellt $R_4$ Wasserstoff dar. In einer anderen bevorzugten Ausführungsform stellt $R_7$ Wasserstoff dar. In einer weiteren bevorzugten Ausführungsform bedeuten $R_5$ und $R_5$ unabhängig voneinander H, F, Cl, Br, OH, SH, $NH_2$, NHOH, $NHNH_2$, Methylamino, Dimethylamino, Benzoylamino, Isobutyrylamino, Methoxy, Ethoxy und Methylthio.

Einige Beispiele für Analoge der Purinreihe sind neben Purin Adenin, N-Methyladenin, N-Benzoyladenin, 2-Methylthioadenin, 2-Aminoadenin, 6-Hydroxypurin, 2-Amino-6-chlorpurin, 2-Amino-6-methylthiopurin, Guanin, N-Isobutyrylguanin. Besonders bevorzugt sind Adenin, 2-Aminoadenin und Guanin, sowie deren basengeschützte Derivate.

Wenn B in Formel I einen analogen Pyrimidinrest darstellt, so handelt es sich bevorzugt um Uracil-, Thymin- und Cytosinreste der Formeln III, IIIa und IIIb,

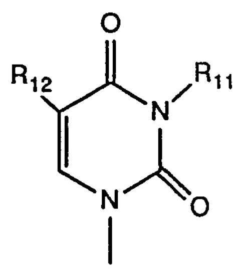

(III),

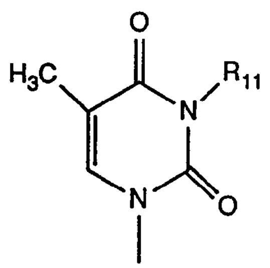

(IIIa)

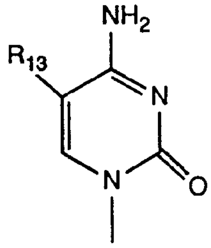

(IIIb),

worin $R_{11}$ H oder $C_1$-$C_4$-Alkyl bedeutet, und $R_{12}$ und $R_{13}$ unabhängig voneinander die zuvor für $R_5$ angegebene Bedeutung haben, einschliesslich der Bevorzugungen, und die Wasserstoffatome der $NH_2$-Gruppe in Formel IIIb mit $C_1$-$C_6$-Alkyl oder Benzoyl substituiert sein können, sowie die Dihydroderivate der Reste der Formeln

III, IIIa und IIIb. Bevorzugt stellt $R_{12}$ H, $C_1$-$C_6$-Alkyl oder Hydroxyalkyl, F, Cl, Br, $NH_2$, Benzoylamino, Mono- oder Di-$C_1$-$C_6$-alkylamino dar und $R_{13}$ stellt bevorzugt H, $C_1$-$C_6$-Alkyl oder -Alkoxy oder -Hydroxyalkyl, F, Cl, Br, $NH_2$, Benzoylamino, Mono- oder Di-$C_1$-$C_6$-alkylamino dar.

$R_{11}$ steht bevorzugt für H oder Methyl. $R_{12}$ bedeutet bevorzugt H, F, Cl, Br, $NH_2$, $NHCH_3$, $N(CH_3)_2$ oder $C_1$-$C_4$-Alkyl. $R_{13}$ stellt bevorzugt H, $C_1$-$C_4$-Alkyl, besonders Methyl, oder $NH_2$, $NHCH_3$ oder $(CH_3)_2N$ dar.

Einige Beispiele für Pyrimidinanaloge sind Uracil, Thymin, Cytosin, 5-Fluoruracil, 5-Chloruracil, 5-Bromuracil, Dihydrouracil und 5-Methylcytosin.

Besonders bevorzugte Verbindungen der Formeln I und Ia sind diejenigen, in denen $R_1$ und $R_2$ für Wasserstoff stehen, A für $-CH_2-$ steht und B Thymin, Adenin, Cytosin oder Guanin bedeutet.

Ein weiterer Gegenstand der Erfindung ist ein Verfahren zur Herstellung von Verbindungen der Formel I, das dadurch gekennzeichnet ist, dass man in einer enantiomeren Verbindung der Formel IV oder IVa oder ihren Racematen

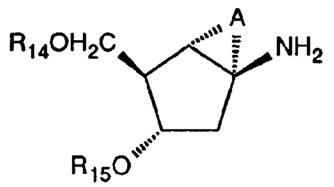 (IV), 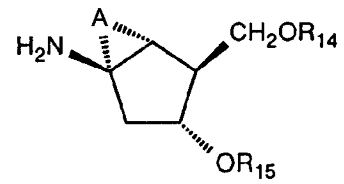 (IVa),

worin A die zuvor angegebene Bedeutung hat, einschliesslich der Bevorzugung, und $R_{14}$ und $R_{15}$ für gleiche oder verschiedene Schutzgruppen stehen, in an sich bekannter Weise in einem inerten Lösungsmittel durch Aufbaureaktionen die $NH_2$-Gruppe in einen Rest B umwandelt, wobei B einen Purin- oder Pyrimidinrest oder ein Analoges davon bedeutet.

Aufbaureaktionen für die auch als Nukleinbasen bezeichneten Reste B sind in der Literatur beschrieben, zum Beispiel von Y. F. Shealy et al. im J. Heterocyclic Chem., 18:383-389 (1981); M. Arita et al. im J. Am. Chem. Soc., 105:4049-4055 (1983); G. V. B. Madhavan et al. im J. Org. Chem., 51:1287-1293 (1986); V. E. Marquez et al. im J. Med. Chem., 31, S. 1687-1694 (1988); J. Balzarini et al. im J. Med. Chem., 32:1861-1865 (1989); M. Koga et al. in Tetrahedron Letters, 31:5861-5864 (1990); M. R. Harnden et al. im J. Med. Chem., 33:187-196 (1990) und A. D. Borthwick et al. in Tetrahedron 48:571-623 (1992) [Übersicht mit weiteren Literaturangaben].

Schutzgruppen sind zuvor erwähnt worden. Die Temperatur in den einzelnen Reaktionsstufen der Aufbaureaktion kann von -80 bis 150 °C, bevorzugt 0 bis 100 °C betragen.

Im allgemeinen verwendet man Lösungsmittel, die protisch und/oder aprotisch, und besonders bevorzugt dipolar sind. Beispiele für Lösungsmittel, die alleine oder als Mischung von mindestens zwei Lösungsmitteln eingesetzt werden können, sind Ether (Dibutylether, Tetrahydrofuran, Dioxan, Diethylenglykoldimethylether, Ethylenglykoldimethyl- oder -diethylether, Diethylenglykoldiethylether, Triethylenglykoldimethylether), halogenierte Kohlenwasserstoffe (Methylenchlorid, Chloroform, 1,2-Dichlorethan, 1,1,1-Trichlorethan, 1,1,2,2-Tetrachlorethan), Carbonsäureester und Lactone (Essigsäureethylester, Propionsäuremethylester, Benzoesäureethylester, 2-Methoxyethylacetat, Methoxymethylacetat, $\gamma$-Butyrolacton, $\delta$-Valerolacton, Pivalolacton), Carbonsäureamide und Lactame (N,N-Dimethylformamid, N,N-Diethylformamid, N,N-Dimethylacetamid, Tetramethylharnstoff, Hexamethylphosphorsäuretriamid, $\gamma$-Butyrolactam, $\varepsilon$-Caprolactam, N-Methylpyrrolidon, N-Acetylpyrrolidon, N-Methylcaprolactam), Sulfoxide (Dimethylsulfoxid), Sulfone (Dimethylsulfon, Diethylsulfon, Trimethylensulfon, Tetramethylensulfon), tertiäre Amine (Triethylamin, N-Methylpiperidin, N-Methylmorpholin), aromatische Kohlenwasserstoffe wie zum Beispiel Benzol oder substituierte Benzole (Chlorbenzol, o-Dichlorbenzol, 1,2,4-Trichlorbenzol, Nitrobenzol, Toluol, Xylol) und Nitrile (Acetonitril, Propionitril, Benzonitril, Phenylacetonitril), sowie aliphatische oder cycloaliphatische Kohlenwasserstoffe (Pentan, Petrolether, Hexan, Cyclohexan und Methylcyclohexan).

Die Verbindungen der Formeln IV und IVa mit den vorstehenden Substituentendefinitionen sind neu und stellen zusammen mit den Verbindungen der Formeln IV und IVa, worin $R_{14}$ und $R_{15}$ für H stehen, einen weiteren Gegenstand der Erfindung dar. Ein weiterer Gegenstand der Erfindung sind somit die enantiomeren Verbindungen der Formeln IV und IVa und deren Racemate

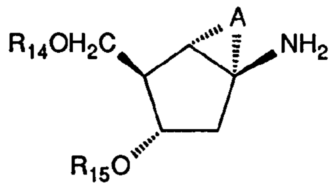 (IV), 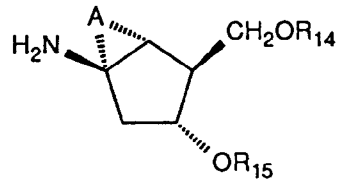 (IVa),

worin A für $-CH_2-$ oder $-CH_2CH_2-$ steht und $R_{14}$ und $R_{15}$ unabhängig voneinander H oder gleiche oder verschiedene Schutzgruppen bedeuten. Für A, $R_{14}$ und $R_{15}$ gelten die zuvor angegebenen Bevorzugungen. Die Verbindungen der Formel IV sind bevorzugt.

Die Verbindungen der Formeln IV und IVa können zum Beispiel nach dem folgenden neuen Verfahren hergestellt werden, das einen weiteren Gegenstand der Erfindung darstellt.

Die Umsetzung der von H.-J. Gais et al. in Liebigs Ann. Chem., 1179-1212 (1986) beschriebenen Verbindung der Formel A

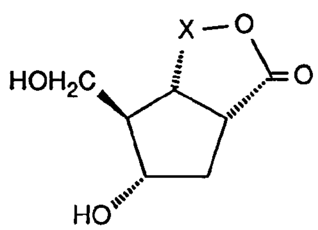 (A),

worin X für die Gruppe $-CH_2-$ steht, mit einem Trialkylsilylhalogenid wie zum Beispiel einem Chlorid, Bromid oder Iodid, zum Beispiel Trimethylsilylchlorid oder -bromid, in alkoholischer (zum Beispiel methanolischer) Lösung gegebenenfalls in Gegenwart von wasserfreien Metallhalogeniden wie zum Beispiel Zinkdichlorid oder -dibromid führt zu Verbindungen der Formel B

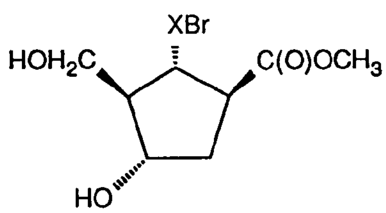 (B).

Aus den hydroxylgeschützten Verbindungen der Formel B können hydroxylgeschützte Verbindungen der Formel B' mit X gleich $-CH_2CH_2-$ erhalten werden, indem man sie zum Beispiel in eine Grignard-Verbindung überführt (zum Beispiel mit Magnesium), diese Grignard-Verbindung durch Reaktion mit Formaldehyd und anschliessender Hydrolyse in die entsprechende β-Hydroxyethyl-Verbindung umwandelt, und diese Hydroxylgruppe anschliessend mit einem Halogenierungsreagenz, zum Beispiel HCl, HBr, $SOCl_2$, $CCl_4$/Triphenylphosphin oder $CBr_4$/Triphenylphosphin umsetzt.

Die Verbindungen der Formel B können in bekannter Weise in hydroxylgeschützte Derivate der Formel C übergeführt werden,

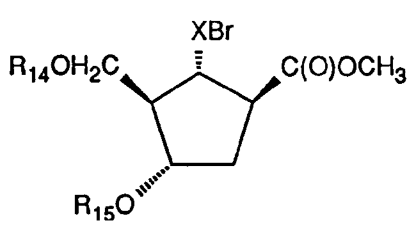 (C),

worin $R_{14}$ und $R_{15}$ für gleiche oder verschiedene Schutzgruppen oder $R_{14}$ und $R_{15}$ zusammen für eine Schutz-

gruppe stehen, zum Beispiel für t-Butyl-dimethylsilyl, und X $-CH_2-$ bedeuten. Die Verbindungen der Formel C umfassen auch die Verbindungen der Formel B', wobei X dann $-CH_2CH_2-$ bedeutet.

Die Verbindungen der Formel C (beziehungsweise B' mit X gleich $-CH_2-CH_2-$) können durch Einwirkung starker Basen (zum Beispiel von Alkalimetallalkoholaten) zu den Verbindungen der Formel D

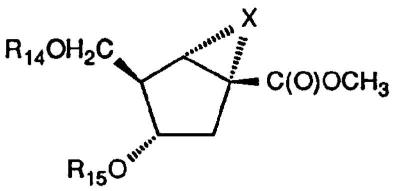

(D)

cyclisiert werden.

Die Verbindungen der Formel D können dann in üblicher Weise zur entsprechenden Carbonsäure verseift werden, die dann in das entsprechende Carbonsäureazid übergeführt wird (zum Beispiel mit Diphenylphosphonylazid). Das Carbonsäureazid wird in Gegenwart eines tertiären Amins, zum Beispiel Triethylamin, zum Isocyanat umgewandelt (Curtius-Umlagerung), das dann mit einem geeigneten Alkohol, zum Beispiel Benzylalkohol, zu einem Urethan der Formel E mit X gleich $-CH_2-$ oder $-CH_2CH_2-$

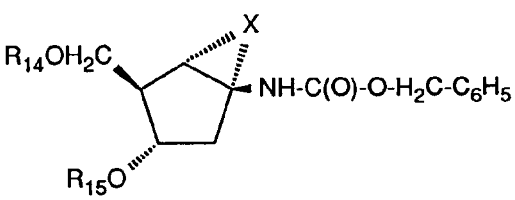

(E)

umgesetzt wird.

Aus den Verbindungen der Formel E können durch Entfernung der Aminoschutzgruppe $-C(O)-O-CH_2-C_6H_5$ die Verbindungen der Formeln IV und IVa zum Beispiel durch katalytische Hydrierung mit Edelmetallkatalysatoren, zum Beispiel Palladium, erhalten werden. Die Verbindungen der Formeln IV und IVa, worin $R_{14}$ und $R_{15}$ für H stehen, erhält man durch Abspaltung der Schutzgruppen vor oder nach der Hydrierung. Die Enantiomeren der Formeln IV und IVa können zum Beispiel durch chromatographische Trennverfahren aus den Racematen erhalten werden. Man kann zu deren Herstellung aber auch von Enantiomeren der Formel A ausgehen, oder man kann eine der Zwischenstufen B bis E chromatographisch trennen und die Umsetzung mit einer der enantiomerenreinen Zwischenstufen fortsetzen.

Von den Verbindungen der Formeln I und Ia werden im allgemeinen vor deren Weiterverarbeitung beziehungsweise zu deren Verwendung als pharmazeutische Wirkstoffe in bekannter Weise die Schutzgruppen abgetrennt, wobei man Verbindungen erhält, in denen $R_1$ und $R_2$ Wasserstoff bedeuten. Aus diesen Verbindungen können Oligonukleotide aufgebaut werden, die auf Grund ihrer Wechselwirkung mit Nukleinsäuren wertvolle biologische Aktivitäten aufweisen, und als pharmazeutische Wirkstoffe oder als Diagnostika verwendet werden können.

Ein weiterer Gegenstand der Erfindung ist die Verwendung der Verbindungen der Formeln I und Ia in Form von Racematen zur Herstellung von Oligonukleotiden, die gleiche oder verschiedene Monomereinheiten von Verbindungen der Formeln I und/oder Ia oder mindestens eine Monomereinheit von Verbindungen der Formeln I und/oder Ia und mindestens eine Monomereinheit von anderen natürlichen oder synthetischen Nukleosiden enthalten, wobei die Oligonukleotide 2 bis 200 Monomereinheiten enthalten. Bevorzugt enthalten die Oligonukleotide 2 bis 100, besonders bevorzugt 2 bis 50, und insbesondere bevorzugt 2 bis 20 Monomereinheiten. Bevorzugt sind Oligonukleotide, die gleiche oder verschiedene und besonders verschiedene Monomereinheiten von Verbindungen der Formeln I und/oder Ia enthalten. Bevorzugt sind ferner zusätzlich Monomereinheiten von synthetischen oder natürlichen Nukleosiden enthalten, die sich von D-Ribose oder 2-Desoxyribose ableiten.

Ein weiterer Gegenstand der Erfindung sind Oligonukleotide der Formel V

$$5'\text{-U-(O-Y-O-V-)}_y\text{O-Y-O-W-3'} \qquad (V),$$

worin U, V und W je für sich gleiche oder verschiedene Reste von natürlichen oder synthetischen Nukleosiden darstellen und mindestens einer der Reste U, V und/oder W einen Rest der Formeln VI und/oder VIa

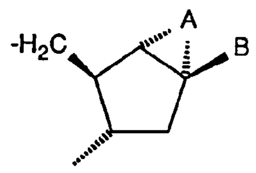 (VI), 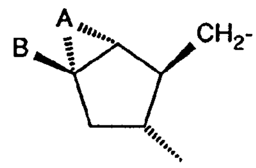 (VIa)

darstellen, und y für eine Zahl von 0 bis 200 steht, Y für eine Nukleotid-Brückengruppe steht, B einen Purin- oder Pyrimidinrest oder ein Analoges davon bedeutet, und A für $-CH_2-$ oder $-CH_2CH_2-$ steht. Für A und B gelten die zuvor für Verbindungen der Formel I angegebenen Bevorzugungen und Beispiele. Eine bevorzugte Brückengruppe ist die in natürlichen Oligonukleotiden vorkommende Gruppe $-P(O)O^{\ominus}-$. Beispiele für weitere Brückengruppen sind $-P(O)S^{\ominus}-$, $-P(S)S^{\ominus}-$, $-P(O)R_{16}-$, $P(O)NR_{17}R_{18}$, oder $-CH_2-$, worin $R_{16}$ H oder $C_1$-$C_6$-Alkyl darstellt und $R_{17}$ und $R_{18}$ unabhängig voneinander die Bedeutung von $R_{16}$ haben. In Formel V steht y bevorzugt für eine Zahl von 0 bis 100, besonders bevorzugt für eine Zahl von 0 bis 50 und insbesondere bevorzugt für eine Zahl von 0 bis 20. Die Reste der Formeln VI und/oder VIa können endständig oder in der Nukleotidsequenz gebunden sein, wobei mehrere, zum Beispiel 2 bis 5 Reste der Formeln VI und/-oder VIa aufeinander folgen können, oder die Reste der Formeln VI und/oder VIa können zwischen Resten von natürlichen oder synthetischen Nukleosiden gebunden sein, oder es können Mischformen dieser Verteilungen in der Nukleotidsequenz vorliegen. Bevorzugt sind insgesamt 4 bis 30 Nukleosideinheiten enthalten und bevorzugt sind 1 bis 12, besonders bevorzugt 1 bis 6 und insbesondere bevorzugt 1 bis 4 Reste der Formeln VI und/oder VIa enthalten.

In einer bevorzugten Ausführungsform enthält das Oligonukleotid der Formel V Reste der Formel VI.

Eine ganz besonders bevorzugte Ausführungsform sind Oligonukleotide der Formel V, worin y eine Zahl von 2 bis 50, bevorzugt 2 bis 30 darstellt, Y für die Gruppe $P(O)O^{\ominus}$-steht, U, V und W je für sich gleiche oder verschiedene Reste eines natürlichen Nukleosids bedeuten und mindestens einer der Reste U, V oder W den Formeln VI und/oder VIa entspricht, A für $-CH_2-$ steht und B der Rest einer natürlichen Nukleosidbase ist. Als natürliche Nukleoside kommen Adenosin, Cytidin, Guanosin, Uridin, 2-Aminoadenin, 5-Methylcytosin, 2'-Desoxyadenosin, 2'-Desoxycytidin, 2'-Desoxyguanosin und Thymidin in Frage. Als natürliche Nukleosidbasen sind besonders Adenin, Cytosin, Guanin, Thymin und Uracil zu nennen. Die Reste der Formeln VI und/oder VIa können endständig oder in der Nukleotidsequenz gebunden sein, wobei mehrere, zum Beispiel 2 bis 5 gleiche oder verschiedene Reste der Formeln VI und/oder VIa, aufeinander folgen können, oder gleiche oder verschiedene Reste der Formeln VI und/oder VIa zwischen Resten von natürlichen Nukleosiden gebunden sind, oder Mischformen dieser Verteilungen in der Nukleotidsequenz vorliegen. In einer anderen bevorzugten Ausführungsform von Oligonukleotiden der Formel V entsprechen sämtliche Reste U, V und W gleichen oder verschiedenen Resten der Formeln VI und/oder VIa, wobei B der Rest einer natürlichen Nukleotidbase ist. Bevorzugt stellt y eine Zahl von 2 bis 20 dar, und bevorzugt sind insgesamt 1 bis 12, besonders bevorzugt 1 bis 6 und insbesondere bevorzugt 1 bis 4 Reste der Formel VIIIb enthalten.

Die Herstellung der erfindungsgemässen Oligonukleotide kann in an sich bekannter Weise nach verschiedenen Verfahren in gegebenenfalls automatisierten und zusammen mit Verfahrensvorschriften käuflichen DNA-Synthesizern erfolgen. Im Falle der Brückengruppe $-P(O)O^{\ominus}-$ kann zum Beispiel das Phosphortriesterverfahren, das Phosphittriesterverfahren oder das H-Phosphonatverfahren angewendet werden, Verfahren, die dem Fachmann geläufig sind. Beim Phosphittriesterverfahren kann man zum Beispiel so vorgehen, dass man die Nukleoside der Formel I, worin $R_1$ und $R_2$ je H bedeuten, in Form ihrer Racemate oder Enantiomeren mit einem Schutzgruppenreagenz, zum Beispiel 4,4'-Dimethoxytriphenylmethyl-chlorid (abgekürzt DMT-Cl) zu einem Nukleosid der Formel F oder F' oder deren Racemate

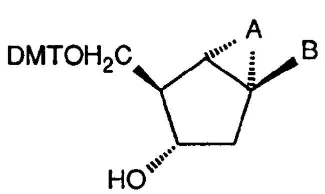 (F), 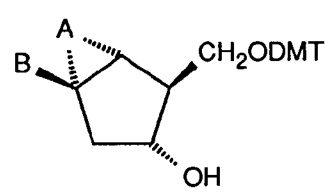 (F')

umsetzt, und die Verbindung der Formel F und/oder F' mit Hilfe eines "linkers", zum Beispiel Bernsteinsäureanhydrid, an ein festes Trägermaterial bindet, zum Beispiel an Controlled Pore Glass (CPG), das langkettige Alkylaminogruppen enthält. In einem separaten Verfahren wird die Hydroxylgruppe der Verbindung der Formel F oder F' derivatisiert, zum Beispiel zu einem Phosphoramidit, indem die Verbindung der Formel F oder F' unter

Verwendung von $ROP[N(i\text{-}Propyl)_2)]_2$ zu einer Verbindung der Formel G und/oder G',

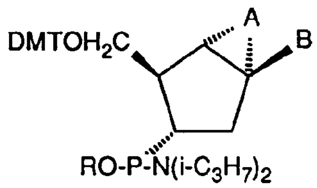 (G), 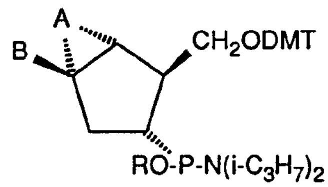 (G')

umgesetzt wird, wobei R zum Beispiel β-Cyanoethyl darstellt.

Nach dem Abspalten der Schutzgruppe des an den Träger gebundenen Materials koppelt man unter Abspaltung von $-N(i\text{-}C_3H_7)_2$ mit der Verbindung der Formel G oder G', blockiert eventuell vorhandene freie Hydroxylgruppen (capping) und oxidiert dann das gebildete Phosphit zum Phosphat. Nach dem Entschützen des Dimeren wiederholt man den Reaktionszyklus mit einer Verbindung der Formel G oder G', bis man ein Oligomer mit der gewünschten Anzahl an Monomereinheiten synthetisiert hat, und löst das Produkt vom Trägermaterial ab. Auf diese Weise erhält man Oligonukleotide, in denen sämtliche Reste U, V und W gemäss Formel V aus Resten der Formeln VI und/oder VIa bestehen. Auf diese Weise sind auch Oligonukleotide mit beliebigen Monomereinheiten in beliebiger Sequenz herstellbar, je nach Verwendung synthetischer, natürlicher und erfindungsgemässer Nukleosidbausteine in den einzelnen Reaktionszyklen.

Die erfindungsgemässen Verbindungen der Formeln I und Ia sowie deren Racemate, worin $R_1$ und $R_2$ je H bedeuten, weisen antivirale und antiproliferative Eigenschaften auf und können demgemäss als Arzneimittel Verwendung finden. Die erfindungsgemässen Oligonukleotide weisen eine überraschend hohe Stabilität gegenüber einem Abbau durch Nukleasen auf. Ferner wird eine sehr gute Paarung mit komplementären Nukleinsäuresträngen, besonders vom RNA-Typ, beobachtet. Die erfindungsgemässen Oligonukleotide eignen sich daher besonders für die Antisense-Technologie, das heisst zur Inhibition der Expression unerwünschter Proteinprodukte durch die Bindung an geeignete komplementäre Nukleotidsequenzen in Nukleinsäuren (siehe EP-A-0 266 099, WO 87/07300 und WO 89/08146). Sie können zur Behandlung von Infektionen und Krankheiten zum Beispiel durch die Blockierung der Expression von bioaktiven Proteinen auf der Stufe der Nukleinsäuren (zum Beispiel Onkogene) eingesetzt werden. Die erfindungsgemässen Oligonukleotide eignen sich auch als Diagnostika und können als Gensonden zum Nachweis von viralen Infektionen oder von genetisch bedingten Krankheiten durch selektive Interaktion auf der Stufe von einzel- oder doppelsträngigen Nukleinsäuren verwendet werden ("gene probes"). Im besonderen - bedingt durch die erhöhte Stabilität gegenüber Nukleasen - ist eine diagnostische Anwendung nicht nur *in vitro* sondern auch *invivo* (zum Beispiel Gewebeproben, Blutplasma und Blutserum) möglich. Solche Verwendungsmöglichkeiten sind zum Beispiel in WO 91/06556 beschrieben.

Ein weiterer Gegenstand der Erfindung betrifft die Verwendung der erfindungsgemässen Oligonukleotide als Diagnostika zum Nachweis von viralen Infektionen oder von genetisch bedingten Krankheiten.

Ein anderer Gegenstand der Erfindung betrifft auch die erfindungsgemässen Nukleoside der Formeln I und/oder Ia sowie die Oligonukleotide der Formel V zur Anwendung in einem therapeutischen Verfahren zur Behandlung von Krankheiten bei Warmblütern einschliesslich des Menschen durch Inaktivierung von Nukleotidsequenzen im Körper. Die Dosierung bei Verabreichung an Warmblüter von etwa 70 kg Körpergewicht kann zum Beispiel 0,01 bis 1000 mg pro Tag betragen. Die Verabreichung erfolgt vorzugsweise in Form pharmazeutischer Präparate parenteral, zum Beispiel intravenös oder intraperitoneal.

Ein weiterer Gegenstand der Erfindung betrifft ein pharmazeutisches Präparat, enthaltend eine wirksame Menge eines Nukleosids der Formeln I und/oder Ia oder eines Oligonukleotids der Formel V alleine oder zusammen mit anderen Wirkstoffen, ein pharmazeutisches Trägermaterial in einer üblichen Menge und gegebenenfalls Hilfsstoffe.

Man kann die pharmakologisch wirksamen erfindungsgemässen Nukleoside und Oligonukleotide in Form von parenteral verabreichbaren Präparaten oder von Infusionslösungen verwenden. Solche Lösungen sind vorzugsweise isotonische wässrige Lösungen oder Suspensionen, wobei diese zum Beispiel bei lyophilisierten Präparaten, welche die Wirksubstanz allein oder zusammen mit einem Trägermaterial, zum Beispiel Mannit, enthalten, vor Gebrauch hergestellt werden können. Die pharmazeutischen Präparate können sterilisiert sein und/oder Hilfsstoffe, zum Beispiel Konservier-, Stabilisier-, Netz- und/oder Emulgiermittel, Löslichkeitsvermittler, Salze zur Regulierung des osmotischen Drucks und/oder Puffer enthalten. Die pharmazeutischen Präparate, die gewünschtenfalls weitere pharmakologisch wirksame Stoffe wie zum Beispiel Antibiotika enthalten können, werden in an sich bekannter Weise, zum Beispiel mittels konventioneller Lösungs- oder Lyophilisierungsverfahren, hergestellt, und enthalten etwa 0,1 % bis 90 %, insbesondere von etwa 0,5 % bis etwa 30

%, zum Beispiel 1 % bis 5 % Aktivstoff(e).

Die nachfolgenden Beispiele erläutern die Erfindung. Den $^{1}H$-NMR-Spektren liegt die Nummerierung der Kohlenstoffatome in den folgenden cyclischen Kohlenstoffgerüsten zu Grunde:

Ausgangsverbindungen:

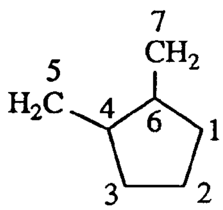

und

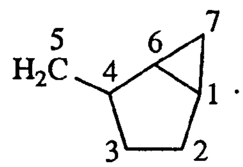

Nukleoside (Beispiele):

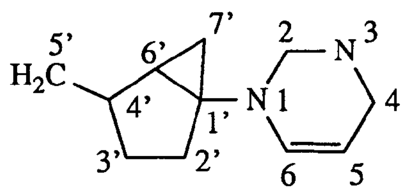

und

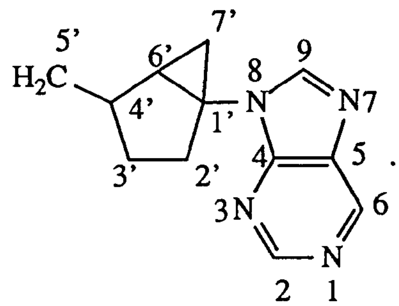

A) Herstellung von Ausgangsverbindungen

Beispiel A1: Herstellung von

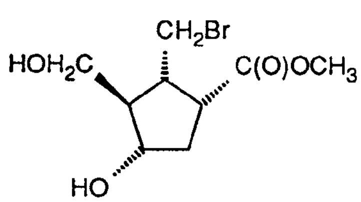

(2).

Zu einer Lösung von 2,0 g (11,6 mmol) der bekannten Verbindung der Formel A in 50 ml Methanol (MeOH) tropft man innerhalb von 5 min bei 0°C 5 ml Trimethylsilylbromid, gibt eine Spatelspitze wasserfreies $ZnBr_2$ zu und rührt anschliessend für 18 h bei 0°C. Das Lösungsmittel wird dann am Rotationsverdampfer entfernt (Badtemperatur < 30°C). Der ölige Rückstand wird in MeOH gelöst, das Lösungsmittel erneut am Rotationsverdampfer entfernt und der Vorgang noch zwei weitere Male wiederholt. Das verbleibende Öl wird im Hochvakuum über NaOH getrocknet und anschliessend durch Flash-Chromatographie an 180 g Kieselgel im Laufmittelsystem Essigsäureethylester/Methanol (9/1) gereinigt. Ausbeute: 2,16 g (70 %) dickflüssiges braunes Öl, das sich bei Raumtemperatur (RT) innerhalb weniger Tage zu zersetzen beginnt, bei -20° aber über längere Zeit lagerbar ist. $^{1}H$-NMR (250 MHz, $CDCl_3$): 4,07 [q, 1H, C(3)H]; 3,68 [s, $OCH_3$].
$^{13}C$-NMR (62,9 MHz, $CDCl_3$): 76,34 [C(3)]; 64,10 [C(5)]; 55,04, 50,84 [CH, $OCH_3$]

Beispiel A2: Herstellung von

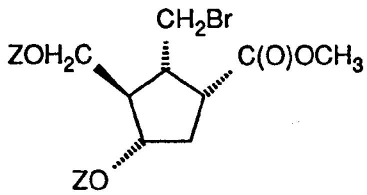

(3).

Z = t-Butyldimethylsilyl-

Eine Lösung von 4,03 g (15,1 mmol) von Verbindung (2) in 50 ml Dimethylformamid (DMF) wird innerhalb von 10 min unter Eiskühlung tropfenweise mit 9,4 ml (45,3 mmol) N-tert-Butyldimethylsilyl-N-methylacetamid versetzt. Das Eisbad wird dann entfernt und 2,5 h bei RT gerührt. Das Reaktionsgemisch wird mit 300 ml Diethylether verdünnt, dreimal mit je 100 ml Eiswasser ausgeschüttelt und die organische Phase getrocknet und am Rotationsverdampfer eingedampft. Das verbleibende gelbe Öl wird an 180 g Kieselgel mit $CH_2Cl_2$ als Laufmittel gereinigt. Man erhält 4,77 g (64 %) eines leicht gelblich gefärbten Öls.

$^1$H-NMR (250 MHz, $CDCl_3$): 3,98 [dd, J = 18 Hz, J = 7 Hz, 1H, C(3)H]; 3,63[m, 5H, $OCH_3$ und C(5)H2]; 0,80 [s, 18 H, $CH_3$(t-Butyl).

$^{13}$C-NMR (62,9 MHz, $CDCl_3$): 71,73 [C(3)]; 60,65 [C(5)]; 53,18 [C(4)]; 36,49 [C(2)]; 33,59 [C(7)]; 24,90 [$CH_3$ (tert-Butyl)]; 24,76 [$CH_3$(tert.-Butyl)].

Beispiel A3:

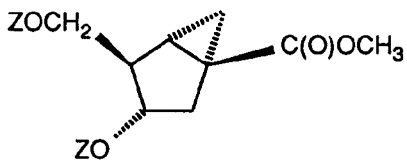

(4).

Z = t-Butyldimethylsilyl-

Zu einer Lösung von 4,72 g (9,52 mmol) von Verbindung (3) in 30 ml tert.-Butanol gibt man bei RT eine Lösung von 1,17 g (10,5 mmol) von Kalium-tert.-Butanolat. Die entstehende Suspension wird 30 min bei RT gerührt und das Reaktionsgemisch dann auf 300 ml Diethylether gegossen. Es wird dreimal mit je 100 ml Eiswasser ausgeschüttelt, die organische Phase getrocknet und das Lösungsmittel am Rotationsverdampfer entfernt. Die Reinigung an 180 g Kieselgel mit $CH_2Cl_2/N(C_2H_5)_3$ (99,5/0,5) als Elutionsmittel ergibt 3,03 g (76 %) Verbindung (4) als farbloses Öl.

$[\alpha]_D^{20}$ = 22,85 (c = 1,204, $CHCl_3$).

$^1$H-NMR (500 MHz, $CDCl_3$): 4,17 [d, J = 6,5 Hz, 1H, C(3)H]; 3,64 [s, 3H, $OCH_3$]; 3,53 [dd, J = 10,0 Hz, J = 5,5 Hz, 1H, C(5)H]; 3,65 [dd, J = 10,0 Hz, J = 7,8 Hz, 1H, C(5)H]; 2,50 [ddd, J = 14,0 Hz, J = 6,5 Hz, J = 1,5 Hz, 1H, C(2)H-β; 1,81 [d, J = 14,0 Hz, 1H, C(2)H-α]; 1,71 [ddd,J = 8,5 Hz, J = 5,0 Hz, J < 1 Hz, 1H, C(6)H]; 1,48 [dd, J = 5,5 Hz, J = 4,0 Hz, 1H, C(7)H-endo]; 1,42 [ddd, J = 8,5 Hz, J = 4,0 Hz, J = 1,5 Hz, 1H, C(7)H-exo]; 0,89 [s, 9 H, $CH_3$ (t-Butyl)]; 0,86 [s, 9 H, $CH_3$ (t-Butyl)].

$^{13}$C-NMR (125,8 MHz, $CDCl_3$): 75,41 [C(3)]; 64,85 [C(5)]; 52,86 [C(4)]; 36,90 [C(2)]; 31,91[C(6)]; 30,84 [C(1)]; 25,92.

Beispiel A4: Herstellung von

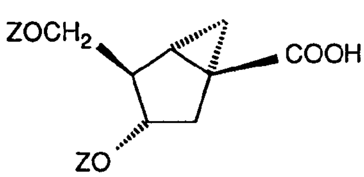

(5).

Z = t-Butyldimethylsilyl

Zu einer Lösung von 2,18 g (5,26 mmol) Verbindung Verbindung (4) in 53 ml Ethanol gibt man unter Eiskühlung 1,03 g (18,44 mmol) fein gepulvertes KOH. Das Eisbad wird dann entfernt, die Mischung 15 min bei RT gerührt und anschliessend 5 h am Rückfluss erhitzt. Das Reaktionsgemisch wird dann am Rotationsverdampfer eingedampft, der Rückstand mit 100 ml Diethylether und 50 ml Eiswasser versetzt und unter heftigem Rühren durch Zugabe von 2N HCl der pH-Wert des Gemischs auf ~ 3 eingestellt (pH Elektrode). Die organische Phase wird abgetrennt und die wässrige Phase noch dreimal mit je 100 ml Ether extrahiert. Die vereinigten organischen Phasen werden getrocknet und am Rotationsverdampfer eingedampft. Der verbleibende semikristalline Rückstand wird durch Flash-Chromatographie an 160 g Kieselgel mit $CH_2Cl_2$/Diethylether (4/l) als Elutionsmittel gereinigt. Ausbeute: 1,63 g (78 %) weisse Kristalle. Smp. 120,1-121,6°C.

$[\alpha]_D^{20}$ = -27,67 (c = 1,095, $CHCl_3$).

$^1$H-NMR (500 MHz, $CDCl_3$): ~ 11 [s, sehr breit, 1H, COOH]; 4,16 [d, J = 6,2 Hz, 1H, C(3)H]; 3,51 [dd, J = 10,0 Hz, J = 6,0 Hz, 1H, C(5)H]; 3,32 [dd, J = 10,0 Hz, J = 8,0 Hz, 1H, C(5)H]; 2,47 [ddd, J = 14 Hz, J = 6,2 Hz, J ~ 1 Hz, 1H, C(2)H-β]; 2,07 [dd, J = 8,0 Hz, J = 6,0 Hz, 1H, C(4)H]; 1,78 [d, J = 14 Hz, 1H, C(2)H-α]; 1,66 [dd, J = 9,0 Hz, J = 5,6 Hz, 1H, C(6)H]; 1,54 [dd, J = 5,6 Hz, J = 4,0 Hz, 1H, C(7)H-endo].

$^{13}$C-NMR (125,8 MHz, $CDCl_3$): 75,36 [C(3)]; 64,78 [C(5)]; 52,87 [C(4)]; 36,37 [C(2)]; 33,24 [C(6)]; 30,72 [C(1)]; 25,96 [$CH_3$ (t-Butyl)]; 25,80 [$CH_3$ (t-Butyl)].

Beispiel A5: Herstellung von

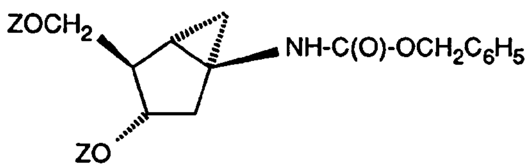

(6).

Z = t-Butyldimethylsilyl-

Zu einer Lösung von 1,41 g (3,53 mmol) Verbindung (5) in 35 ml absolutem Toluol gibt man bei 0°C 882 µl (3,88 mmol) Diphenylphosphorylazid (95 %) und 586 µl (4,24 mmol)

$N(C_2H_5)_3$. Die Lösung wird 1 h bei 0°C und 1,5 h bei RT und 2 h bei 80°C gerührt. Nach dem Abkühlen auf RT werden 804 µl (7,77 mmol) wasserfreier Benzylalkohol sowie eine Spatelspitze Dibutylzinnlaurat zugegeben und die Lösung 2 h bei 80°C und anschliessend noch 15 min bei 100°C gerührt. Das Reaktionsgemisch wird dann auf RT abgekühlt, mit 100 ml Diethylether verdünnt und zweimal mit je 20 ml gesättigter $NaHCO_3$ und zweimal mit je 100 ml Wasser ausgeschüttelt. Nach dem Trocknen über $MgSO_4$ wird die organische Phase am Rotationsverdampfer eingedampft und der verbleibende ölige Rückstand durch Flash-Chromatographie an 80 g Kieselgel mit $CH_2Cl_2$ als Elutionsmittel gereinigt. Es werden 1,51 g (85 %) nicht ganz reine Verbindung (6) als dickflüssiges Öl erhalten.

$[\alpha]_D^{20}$ = +6,52 (c = 1,012, $CHCl_3$).

$^1$H-NMR (500 MHz, $CDCl_3$) (alle Signale sehr breit): 4,13 [m, 1H, C(3)H] 3,85 [m, 1H, C(5)H]; 3,57 [m, 1H, C(5)H]; 2,32 [m, 1H, C(2)H-β]; 1,95 [m, 1H, C(4)H]; 1,90 [d, J = 14 Hz, 1H, C(2)H-α]; 1,13 [m, 1H, C(6)H + C(7)H-endo].

$^{13}$C-NMR (125,8 MHz, $CDCl_3$) (viele Signale sehr breit): 155,93 [C = O (Urethan)];

136,58 [C aromatisch]; 128,54 [C aromatisch]; 128,10 [C aromatisch]; 74,21 [C(3)]; 66,36 [$C_6H_5$-$CH_2$]; 64,63 [C(5)]; 53,41 [C(4)]; 40,42 [C(1)]; 40,04 [C(2)]; 26,89 [C(6)].

Beispiel A6: Herstellung von

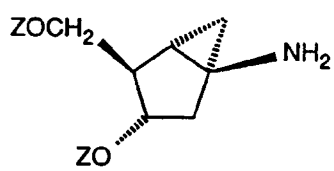

(7).

Z = t-Butyldimethylsilyl-

Verbindung (6) (3,23 g, 6,40 mmol) wird in 65 ml absolutem Toluol gelöst. Nach Zugabe von 650 mg 10 % Pd/C wird für 2 h unter kräftigem Rühren $H_2$ durch das Gemisch geleitet. Der Katalysator wird dann durch Filtration entfernt, mit Essigsäureethylester nachgewaschen und die vereinigten Filtrate werden dreimal mit je 50 ml gesättigtem $NaHCO_3$ extrahiert. Nach dem Trocknen über $MgSO_4$ und Entfernung des Lösungsmittels am Rotationsverdampfer wird die verbleibende farblose Flüssigkeit im Elutionssystem Essigsäureethylester/Methanol (19/1) bis Essigsäureethylester/Methanol (9/1) chromatographiert. Es werden 870 mg (84 %) der Verbindung (7) als farbloses Öl erhalten.

$[\alpha]_D^{20}$ = +10,76 (c = 1,00, $CHCl_3$)

$^1$H-NMR (500 MHz, $CDCl_3$): 4,10 [dd, J = 6,5 Hz, J < 1 Hz, 1H, C(3)H]; 3,57 [dd, J = 10,0 Hz, J = 5,3 Hz, 1H, C(5)H]; 3,38 [dd, J = 10,0 Hz, J = 8,0 Hz, 1H, C(5)H]; 2,02 [ddd, J = 13,0 Hz, J = 6,5 Hz, J = 2,0 Hz, 1H, C(2)H-β]; 1,92 [dd, J = 8,0 Hz, J = 5,3 Hz, 1H, C(4)H]; 1,89 [d, J = 13 Hz, 1H, C(2)H-α]; 1,62 [s, breit, 2H, $NH_2$]; 1,11 [dd (triplettoid), J = 4,5 Hz, J = 4,5 Hz, 1H, C(7)H-endo].

$^{13}$C-NMR (125,8 MHz, $CDCl_3$): 74,99 [C(3)]; 65,45 [C(5)]; 53,53 [C(4)]; 44,86 [C(2)]; 42,67 [C(1)]; 27,67 [C(6)]; 18,46 [C(7)]; 26,02 [$CH_3$ (t-Butyl)]; 25,81 [$CH_3$ (t-Butyl)].

B) Herstellung von Nukleosid-analogen

Beispiel B1: Herstellung von

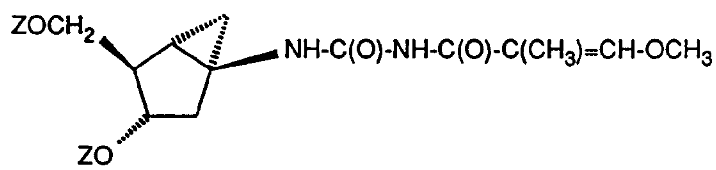

(8).

Z = t-Butyldimethylsilyl-

Eine Lösung von 770 mg (2,08 mmol) Verbindung (7) in 10 ml $CH_2Cl_2$ wird auf -60°C gekühlt und bei dieser Temperatur werden 387 mg (2,70 mmol) β-Methoxy-α-methyl- acryloylisocyanat langsam zugetropft. Das Kältebad wird entfernt und die Mischung auf RT erwärmt. Nach 18-stündigem Stehen bei RT wird mit Diethylether verdünnt, dreimal mit je 20 ml gesattigtem $NaHCO_3$ ausgeschüttelt, über $MgSO_4$ getrocknet und eingedampft. Die Flash-Chromatographie an 80 g Kieselgel mit $CH_2Cl_2$/Diethylether als Elu-tionsmittel ergeben 898 mg (85 %) an Verbindung (8) als dickflüssiges Öl.

$[\alpha]_D^{20}$ = -22,69 (c = 1,04, $CHCl_3$).

$^1$H-NMR (500 MHz, $CDCl_3$): 8,79 [s, 1H, NH(Imid)]; 7,36 [s, 1H, NH(Amid)]; 7,30 [d, J=1,1Hz, 1H,C=CH];4,20[dd,J=6,5Hz, $^4J_{W3,6}$ < 1 Hz, 1H, C(3)H]; 3,67 [dd, J = 10,0 Hz, J = 5,5 Hz, 1H, C(5)H]; 2,40 [ddd, J = 13 Hz, J = 6,5 Hz, J = 2,0 Hz, 1H, C(2)H-β]; 2,01 [dd, J = 10 Hz, J = 5,5 Hz, 1H, C(4)H]; 1,95 [d, J = 13,0 Hz, 1H, C(2)H-α].

$^{13}$C-NMR (125,8 MHz, $CDCl_3$): 169,34 [C = O Amid]; 158,49 [= CH($OCH_3$)]; 154,49 [C = O Harnstoff]; 107,11 [-C($CH_3$)=].

Beispiel B2: Herstellung von

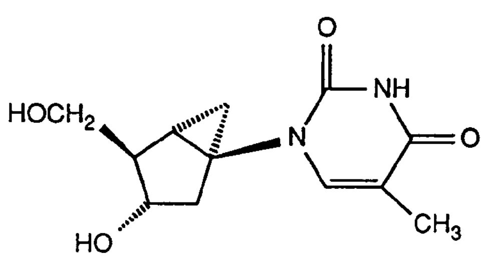

(9).

Eine Lösung von 2,57 g (5,02 mmol) Verbindung (8) in 58 ml Ethanol und 6,46 ml 2N wässriger HCl wird 10 h zum Rückfluss erhitzt. Das Lösungsmittel wird dann am Rotatationsverdampfer entfernt und das verbleibende Öl dreimal mit Ethanol am Rotationsverdampfer behandelt. Der hierbei erhaltene Schaum wird bei 40°C in 10 ml Isopropanol gelöst und es wird unter Rühren 10 ml Pentan zugegeben. Nach 7-stündigem Stehen bei RT und 2 h bei -20°C erhält man 1,01 g (80 %) kristalline Verbindung (9). Smp. 206-206,4°C.

$^1$H-NMR (500 MHz, DMSO-$d_6$): 11,13 [s, 1H, NH]; 7,46 [d, J = 1,2 Hz, 1H, C(6)H]; 4,71 [t, J = 5,1 Hz, C(5)$H_2$OH]; 4,62 [d, J = 3,0 Hz, C(3)HOH]; 4,02 [dd,$^3$J = 6,5 Hz, $^4J_{W3'6'}$ = 2,0 Hz, 1H, C(3)H]; 3,50 [m, 2H, C(5)$H_2$]; 2,10

[ddd, ${}^3J_{2'\beta 2'\alpha}$ = 13 Hz, ${}^3J_{2'\beta 3'}$' 6,5 Hz, ${}^4J_{W2'\beta 7'-exo}$ =2,0 Hz, 1H, C(2')H-β]; 1,92 [d, J = 13 Hz, 1H, C(2')H-α]; 1,87 [t, J = 6 Hz, 1H, C(4')H]; 1,72 [d, J = 1,2 Hz, 3H, C(5)$CH_3$]; 1,55 [ddd, J = 10 Hz, J = 5 Hz, J = 1 Hz, 1H, C(6')H]; 1,33 [dd (triplettoid), J = 5 Hz, J = 5 Hz, 1H, C(7')H-endo]; 1,00 [ddd, J = 10 Hz, J = 5 Hz, J = 2 Hz, 1H, C(7')H-exo].

Beispiel B3: Herstellung von

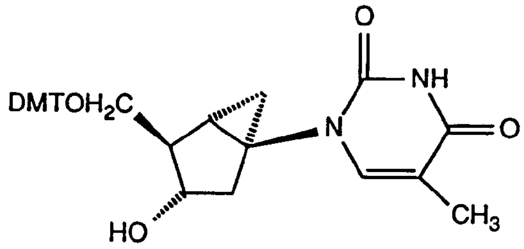

(10).

DMT = 4,4'-Dimethoxytrityl

Zu einer Lösung von 302 mg (1,2 mmol) Verbindung (9) in 12 ml absolutem Pyridin gibt man 488 mg (1,44 mmol) 4,4'-Dimethoxytritylchlorid, 233 µl (1,68 mmol) $N(C_2H_5)_3$ und eine Spatelspitze N,N-Dimethyl-4-aminopyridin. Es wird 3 h bei RT gerührt und das Reaktionsgemisch dann auf 50 ml Diethylether und 50 ml Eiswasser gegossen. Die organische Phase wird abgetrennt und die wässrige Phase noch dreimal mit je 50 ml Diethylether extrahiert. Das nach Trocknen und Eindampfen der vereinigten organischen Extrakte verbleibende Öl wird durch Flash-Chromatographie an 80 g Kieselgel mit Essigsäureethylester/$N(C_2H_5)_3$ (99/1) gereinigt. Nach erneuter Chromatographie an 80 g Kieselgel mit $CH_2Cl_2$/Methanol/$N(C_2H_5)_3$ (19/1/0,02) werden 542 mg (82 %) der Titelverbindung als schwach gelblich gefärbter Schaum erhalten.

${}^1$H-NMR (250 MHz, $CDCl_3$): 8,45 [s, breit, 1H, NH]; 7,45-7,15 [m, 9H, H aromat.]; 7,10 [s, 1H, C(6)H]; 6,82 [d, J = 7,5 Hz, 4H, H aromat.]; 4,38 [d, J = 6,5 Hz, 1H, C(3')H]; 3,78 s, 6H, $OCH_3$]; 3,49 [dd, J = 12,5 Hz, J = 5,5 HZ, 1H, C(5')H]; 3,25 [dd, J = 12,5 Hz, J = 6,5 Hz, 1H, C(5')H]; 2,40-2,10 [m, 3H, C(2')H-α + -β, C(4')H]; 1,79 [s, breit, 1H, OH]; 1,65-1,55 [m, 5H, C(6)-$CH_3$ + C(6')H + C(7')H-endo]; 1,12 [m, 1H, C(7')H-exo].

Beispiel B4: Herstellung von

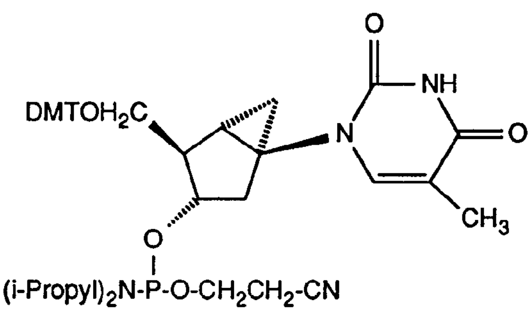

(11).

DMT = 4,4'-Dimethoxytrityl

Zu einer Lösung von 200 mg (1,17 mmol) Diisopropylammoniumtetrazolid und 324 mg (1,08 mmol) 2-Cyanoethoxy-bis(diisopropylamino)phosphin in 6 ml absolutem $CH_2Cl_2$ tropft man innerhalb von 2 min eine Lösung von 542 mg (0,98 mmol) von Verbindung (10) in 6 ml absolutem $CH_2Cl_2$. Die Mischung wird 3 h bei RT gerührt, dann auf ein Gemisch aus 70 ml gesättigter $NaHCO_3$ und 70 ml $CH_2Cl_2$ gegossen, die organische Phase abgetrennt und die wässrige Phase noch dreimal mit je 50 ml $CH_2Cl_2$ extrahiert. Die vereinigten organischen Extrakte werden getrocknet ($MgSO_4$), am Rotationsverdampfer eingedampft und der verbleibende Rückstand durch Flash-Chromatographie an 80 g Kieselgel gereinigt [Essigsäureethylester/Toluol/$N(C_2H_5)_3$ (1/1/0,02)]. Die so gereinigte Titelverbindung wird in 2 ml absolutem $CH_2Cl_2$ gelöst und diese Lösung unter Rühren in 80 ml Pentan eingetropft. Nach Filtration und Trocknen erhält man 544 mg (74 %) von Verbindung (11) [Diastereomerengemisch] als amorphes, elektrostatisch stark aufgeladenes weisses Pulver.

${}^1$H-NMR (500 MHz, $CDCl_3$): 7,45-7,10 [m, 11H]; 6,88-6,78 [m, 4H]; 4,60 [m, breit, 1H, C(3')H]; 3,80-3,58 [m, inklusive Singlett bei 3,80 ($OCH_3$), 5H]; 3,65-3,60 [m, 2H]; 3,38 [m, 1H]; 3,28 [m, 1H]; 2,60-2,30 [m, 5H]; 1,65-1,50 [m, 5H]; 1,20-1,08 [m, 13H];

$^{31}P$-NMR ($CDCl_3$): 147,80

Beispiel B5: Herstellung von

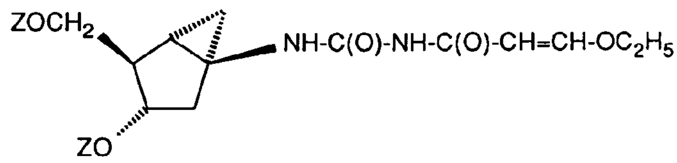

(12).

Z = t-Butyldimethylsilyl-

Eine Lösung von 2,25 g (6,06 mmol) Verbindung (7) in 20 ml absolutem $CH_2Cl_2$ werden auf -60°C gekühlt und bei dieser Temperatur 1,11 g (7,88 mmol) β-Ethoxyacryloylisocyanat zugegeben. Das Kältebad wird dann entfernt und die Mischung auf RT erwärmt. Nach 30 min bei RT wird mit Diethylether verdünnt, dreimal mit je 50 ml gesättigter $NaHCO_3$ ausgeschüttelt, die organische Phase getrocknet und eingedampft. Die Reinigung des Rückstandes an 100 g Kieselgel mit $CH_2Cl$/Diethylether/$N(C_2H_5)_3$ (19/1/0,2) ergibt 2,95 g (95 %) der Titelverbindung als weissen Schaum.

$^1H$-NMR (250 MHz, $CDCl_3$) 2 Isomere (~ 10/1), nur Hauptisomer angegeben: 9,96 [s, 1H, NH (Imid)]; 8,86 [s, 1H, NH (Amid)]; 7,58 [d, J = 12,0 Hz, 1H, =CH-O-]; 5,28 [d, J = 12,0 Hz, 1H, (O)C-CH=]; 4,12 [d, J = 5,0 Hz, 1H, C(3)H]; 3,90 [m, 2H, C(5')H]; 3,55 [m, 2H, $OCH_2CH_3$]; 2,33 [dd, J = 13 Hz, J = 6,0 Hz, 1H, C(2')H-β]; 1,93 [m, 2H, C(2)H-α + C(4)H]; 1,40-1,15 [m, 5H, $OCH_2CH_3$ + C(6)H + C(7)H-endo]; 0,84 (s, breit, $CH_3$ (tert-Butyl), überlagert C(7)H-exo] ; 0,00 [s, breit, Si-$CH_3$].

Beispiel B6: Herstellung von

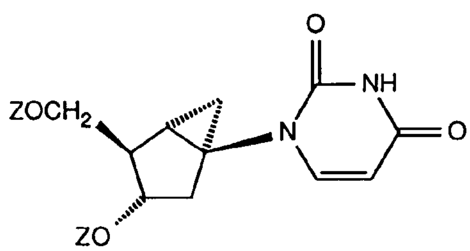

(13).

Z = t-Butyldimethylsilyl-

Eine Lösung von 2,95 g (5,77 mmol) Verbindung (12) in 104 ml Ethanol und 11,5 ml 2N wässriger HCl wird 7 h am Rückfluss erhitzt. Das Lösungsmittel wird dann am Rotationsverdampfer entfernt, der verbleibende Rückstand viermal mit Ethanol am Rotationsverdampfer behandelt, im Hochvakuum getrocknet und dann in 50 ml DMF wiederaufgelöst. Zu dieser Lösung gibt man 1,18 g (17,31 mmol) Imidazol und 2,08 g (13,85 mmol) tert-Butyldimethylsilylchlorid sowie eine Spatelspitze N,N-Dimethyl-4-aminopyridin. Nach 18-stündigem Rühren bei RT wird mit 100 ml Diethylether verdünnt, dreimal mit je 50 ml Eiswasser ausgeschüttelt, über $MgSO_4$ getrocknet und eingedampft. Die Reinigung an 200 g Kieselgel mit Diethylether als Elutionsmittel ergibt 2,35 g (87 %) der Titelverbindung als weissen Schaum.

$^1H$-NMR (250 MHz, $CDCl_3$): 9,43 [s, breit, 1H, NH]; 7,35 [d, J = 8,0 Hz, C(6)H]; 5,57 [d, J = 8,0 Hz, 1H, C(5)H]; 4,15 [d, J = 5,5 Hz, 1H, C(3')H]; 3,68 [d, J = 4 Hz, 2H, C(5')$H_2$]; 2,24 [m, 1H, C(2')H-β]; 1,97 [m, 2H, C(2')H-α + C(4')H]; 1,48 [m, 2H, C(6')H + C(7')H-endo]; 0,82, 0,78 [2 x s, $CH_3$ (tert-Butyl), überlagern C(7')H-exo]; 0,00, -0,08 [2 x s, Si-$CH_3$].

Beispiel B7: Herstellung von

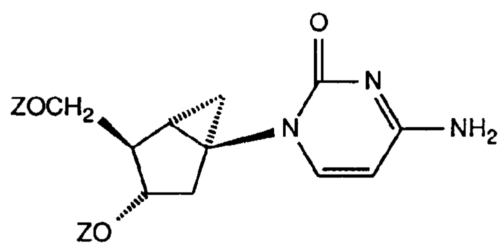

(14).

Z = t-Butyldimethylsilyl-

Zu einer Lösung von 1,865 g (4 mmol) Verbindung (13), 12,8 ml (92,5 mmol) $N(C_2H_5)_3$ sowie 6,22 g (90 mmol) 1,2,4-Triazol in 40 ml absolutem $CH_3CN$ werden innerhalb von 10 min 891 µl (9,76 mmol) frisch destilliertes $POCl_3$ getropft. Es wird 1,5 h bei RT gerührt, dann auf ein Gemisch aus 500 ml $CH_2Cl_2$, 50 ml $N(C_2H_5)_3$ und 150 ml gesättigter $NaHCO_3$ gegossen, die organische Phase abgetrennt und die wässrige Phase noch zweimal mit je 100 ml $CH_2Cl_2$ extrahiert. Nach Trocknen und Eindampfen der organischen Extrakte wird der Rückstand über 180 g Kieselgel filtriert (Diethylether); das so erhaltene Tetrazolid (weisser Schaum) wird in 48 ml Dioxan gelöst, 16 ml konzentrierter Ammoniak zugegeben und 18 h bei 40°C gerührt. Nach Entfernung der Lösungsmittel am Rotationsverdampfer wird der Rückstand zwischen $CH_2Cl_2$ und Wasser verteilt, die organische Phase abgetrennt und die wässrige Phase noch zweimal mit je 100 ml $CH_2Cl_2$ extrahiert. Nach dem Trocknen und Eindampfen der vereinigten organischen Extrakte wird der verbleibende Rückstand an 180 g Kieselgel mit Methanol/Essigsäureethylester als Laufmittel flash-chromatographiert. Man erhält 1,37 g (73 %) der Titelverbindung als amorphen Festkörper.

$^1$H-NMR (250 MHz, $CDCl_3$): 7,31 [d, J = 5,5 Hz, 1H, C(6)H]; 5,68 [d, J = 5,5 Hz, 1H, C(5)H]; 4,16 [d, J = 5,5 Hz, 1H, C(3')H]; 3,72 [m, 2H, C(5')$H_2$]; 2,33 [dd, J = 14,0 Hz, J = 6,0 Hz, 1H, C(2')H-β]; 1,99 [dd (triplettoid), J = 6,5 Hz, J = 6,5 Hz, 1H, C(4')H]; 1,92 [d, J = 14,0 Hz, C(2')H-α]; 1,40 [m, 2H, C(6')H + C(7')H-endo]; 0,92 [m, C(7')H-exo]; 0,82, 0,79 [2 überlappende s, $CH_3$ (tert-Butyl)]; 0,00, -0,06 [2 überlappende s, Si-$CH_3$].

Beispiel B8: Herstellung von

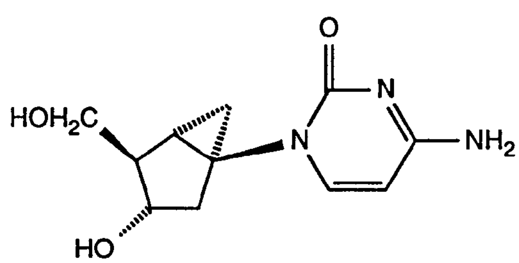

(15).

Zu einer Lösung von 200 mg (0,43 mmol) Verbindung (14) in 5 ml Tetrahydrofuran (THF) gibt man 1,71 ml (1,71 mmol) einer 1M Lösung von Tetrabutylammoniumfluorid in THF. Nach 2 h bei RT wird die Reaktionsmischung am Rotationsverdampfer eingedampft und der Rückstand an 180 g Kieselgel mit Ethanol als Elutionsmittel gereinigt. Man erhält so 80 mg der Titelverbindung als noch leicht mit Tetrabutylammoniumfluorid verunreinigten Festkörper. Umkristallisation aus 5 ml Isopropanol ergibt 52,2 mg (51 %) reine Verbindung (15).

$^1$H-NMR (250 MHz, $CDCl_3$): 7,61 [d, J = 7,5, 1H, C(6)H]; 5,80 [d, J = 7,5 Hz, 1H, C(5)H]; 4,20 [d, J = 7,0 Hz, C(3')H]; 3,77 [dd, J = 11,0 Hz, J = 4,0 Hz, 1H, C(5')H]; 3,64 [J = 11,0 Hz, J = 4,5 Hz, 1H, C(5')H]; 2,37 [ddd, J = 13,5 Hz, J = 7,0 Hz, J = 2,5 Hz, 1H, C(2')H-β]; 2,03 [dd (triplettoid), J = 4,5 Hz, J = 4,5 Hz, 1H, C(4')H]; 1,98 [d, J = 13,5 Hz, 1H, C(2')H-α]; 1,65 [ddd, J = 9,5 Hz, J = 5,0 Hz, J ~ 1 Hz, 1H, C(6')H]; 1,40 [dd (triplettoid), J = 5,0 Hz, J = 5,0 Hz, 1H, C(7')H-endo]; 1,05 [ddd, J = 9,5 Hz, J = 5,0 Hz, J = 2,5 Hz, 1H, C(7')H-exo].

Beispiel B9: Herstellung von

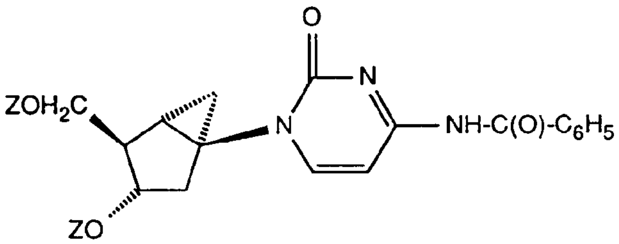

(16).

Z = t-Butyldimethylsilyl-

Zu einer Lösung von 630 mg (1,35 mmol) Verbindung (15) und 279 µl (2,02 mmol) $(C_2H_5)_3N$ in 20 ml absolutem Diethylether tropft man 188 µl (1,62 mmol) Benzoylchlorid und gibt anschliessend noch eine Spatelspitze N,N-Dimethyl-4-aminopyridin zu. Nach 1 h Rühren bei RT wird 1 ml Methanol zugegeben und das Reaktionsgemisch auf eine Mischung aus 50 ml Diethylether und 20 ml gesättigtes $NaHCO_3$ gegossen. Die organische Phase wird abgetrennt, zweimal mit je 20 ml $H_2O$ gewaschen, getrocknet und eingedampft. Die Filtration über 80 g Kieselgel zunächst mit $CH_2Cl_2$ (2 Fraktionen zu 80 ml) und anschliessend mit Diethylether als Elutionsmittel ergeben 729 mg (95 %) der Titelverbindung als weissen Schaum.

$^1$H-NMR (250 MHz, $CDCl_3$): 9,05 [sehr breit, NH]; 7,90-7,80 [m, 3H, 2H aromat. + C(6)H]; 7,60-7,30 [m, 4H, 3H aromat. + C(5)H]; 4,23 [d, J = 5,5 Hz, 1H, C(3')H]; 3,78 [m, 2H, C(5')$H_2$]; 2,40 [dd, J = 12,5 Hz, J = 6,5 Hz, 1H, C(2')H-β]; 2,08 [m, 1H, C(2')H-α + C(4')H]; 1,61 [dd (triplettoid), J = 5,0 Hz, J = 5,0 Hz, 1H, C(6')H]; 1,53 [dd, J = 9 Hz, J = 5,0 Hz, 1H, C(7')H-endo]; 1,10 [m, 1H, C(7')H-exo]; 0,88, 0,84 [2 x s, $CH_3$ (tert-Butyl)]; 0,08, 0,00 [2 x s, Si-$CH_3$].

Beispiel B10: Herstellung von

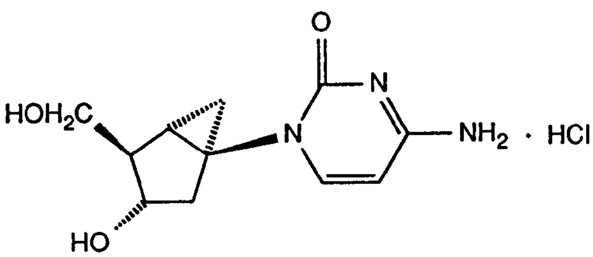

(15a).

Eine Lösung von 653 mg (1,11 mmol von Verbindung (16) in 18 ml Ethanol und 2 ml 2N HCl wird 2 h am Rückfluss erhitzt. Das Lösungsmittel wird dann am Rotationsverdampfer abgezogen, der Rückstand dreimal mit Ethanol am Rotationsverdampfer behandelt, dann in 30 ml heissem Methanol gelöst, sehr wenig Diethylether zugegeben und die Mischung 2 Tage bei RT stehengelassen. Auf diese Weise erhält man 224 mg (72 %) des Hydrochlorids von Verbindung (15) als kristallinen Festkörper (Abspaltung aller Schutzgruppen).

$^1$H-NMR (250 MHz, $D_2O$): 8,03 [d, J = 7,0 Hz, 1H, C(6)H]; 6,23 [d, J = 7,0 Hz, 1H, C(5)H]; 4,37 [d, J = 5,5 Hz, 1H, C(3')H]; 3,82 [d, J = 4,0 Hz, 2H, C(5')$H_2$]; 2,55 [dd, J = 13,5 Hz, J = 7,0 Hz, 1H, C(2')H-β]; 2,23 ["t", 2H, C(2')H-α + C(4')H]; 1,98 [dd, J = 10,0 Hz, J = 4,0 Hz, 1H, C(6')H]; 1,50 [dd (triplettoid), J = 7,0 Hz, J = 7,0 Hz, 1H, C(7')H-endo]; 1,42 [m, 1H, C(7')H-exo].

Beispiel B11: Herstellung von

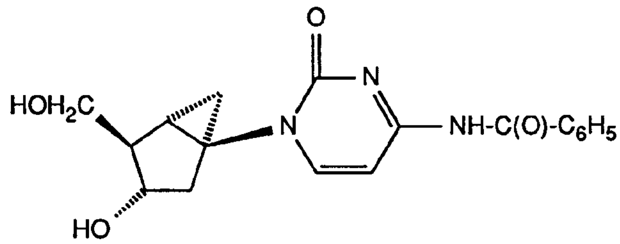

(17).

Zu einer Lösung von 729 mg (1,28 mmol) Verbindung (16) in 20 ml THF gibt man 5,12 ml (5,12 mmol) einer 1M Lösung von Tetrabutylammoniumfluorid (TBAF) in THF. Nach 18 h bei RT werden weitere 5,12 ml der TBAF-Lösung zugefügt, und nach insgesamt 20 h bei RT wird noch 1 h auf 40°C erhitzt. Das Lösungsmittel wird dann am Rotationsverdampfer entfernt und der Rückstand durch Flash-Chromatographie an 180 g Kieselgel mit Essigsäureethylester/Methanol als Elutionsmittel gereinigt. Die produkthaltigen Fraktionen werden zur Trockene eingedampft und der Rückstand aus 10 ml Isopropanol umkristallisiert. Man erhält so 230 mg der reinen Titelverbindung. Der nach Eindampfen der Mutterlauge erhaltene Rückstand wird nochmals an 80 g Kieselgel (Essigsäureethylester/Methanol 4/1) flash-chromatographiert. Die produkthaltigen Fraktionen werden wiederum vereinigt und eingedampft. Der Rückstand wird in 1 ml Isopropanol aufgenommen, und nach Zugabe von 5 ml Ether und einem Impfkristall die Mischung für 15 min auf 0°C gekühlt. Die Filtration ergibt zusätzlich 116 mg an (17). Gesamtausbeute: 346 mg (79 %).

$^1$H-NMR (250 MHz, $CD_3OD/D_2O$): 8,19 [d, J = 6,0 Hz, 1H, C(6)H]; 7,97 [d, J = 6,0 Hz, 2H, H aromat.]; 7,65-7,45 [m, 4H, 3H aromat. + C(5)H]; 4,30 [d, J = 5,5 Hz, 1H, C(3')H]; 3,85 [dd, J = 11,0 Hz, J = 4,0 Hz, 1H, C(5')H]; 3,77 [dd, J = 11,0 Hz, J = 5,5 Hz, 1H, C(5')H]; 2,52 [ddd, J = 13 Hz, J = 6,5 Hz, J ~ 2 Hz, 1H, C(2')H-β]; 2,15 [m, 2H, C(2')H-α + C(4')H]; 1,80 [dd, J = 16,5 Hz, J = 4,0 Hz, 1H, C(6')H]; 1,59 [dd (triplettoid), J = 5,0 Hz, J = 5,0 Hz, 1H, C(7')H-endo]; 1,23 [m, 1H, C(7')H-exo].

Beispiel B12: Herstellung von

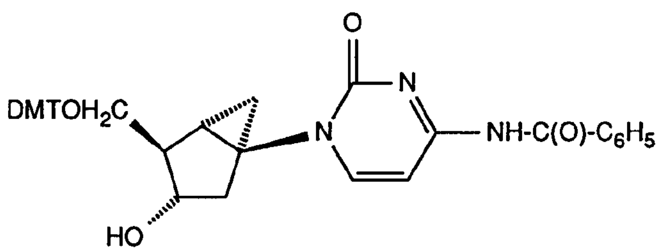

(18).

DMT = 4,4'-Dimethoxytrityl

Analog zu Beispiel B3 erhält man aus 346 mg (1,01 mmol) Verbindung (17) und 410 mg (1,21 mmol) 4,4'-Dimethoxytritylchlorid 563 mg (88 %) der Titelverbindung als ganz schwach blassgelb gefärbten Schaum.

$^1$H-NMR (250 MHz, $CDCl_3$): 8,95 (sehr breit, NH); 7,90 [d, J = 7,5 Hz, 2H, H aromat. o zu CONH]; 7,60-7,15 [m, 14H]; 6,85 [d, J = 9,0 Hz, 4H, H aromat. o zu $OCH_3$]; 4,35 [d, J = 5,5 Hz, 1H, C(3')H]; 3,80 [s, 6H, $OCH_3$]; 3,40-3,10 [m, 3H, C(5')$H_2$ + OH]; 2,49 [d, J = 13,5 Hz, 1H]; 2,35-2,15 [m, 2H]; 1,80-1,65 [m, 2H]; 1,11 [m, 1H].

Beispiel B13: Herstellung von

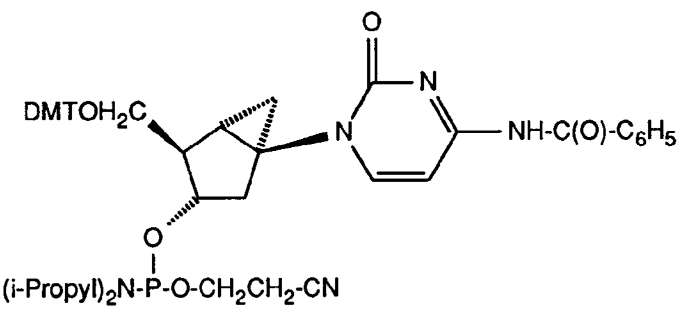

(19).

DMT = 4,4'-Dimethoxytrityl

Analog zu Beispiel B4 erhält man aus 563 mg (0,876 mmol) von Verbindung (18) und 291 mg (0,964 mmol) 2-Cyanoethoxy-bis(diisopropylamino)phosphin 646 mg (86 %) der Titelverbindung als Diastereomerengemisch.

$^{1}$H-NMR (250 MHz, $CDCl_3$): 8,60 (sehr breit, NH); 7,89 [d, J = 7,0 Hz, 2H, H aromat. o zu CONH]; 7,60-7,20 [m, 14H]; 6,86 [d, J = 8,0 Hz, 4H, H aromat. o zu $OCH_3$]; 4,20 [m, breit, 1H, C(3')H]; 3,85-3,65, [m inklusive s bei 3,81 ($OCH_3$), 8H]; 3,65-3,45 [m, 2H] 3,45-3,25 [m, 2H]; 2,65-2,25 [m, 5H]; 1,62-1,58 [m, 2H]; 1,21-1,10 [m, 13H].

$^{31}$P-NMR ($CDCl_3$): 147,80; 147,72.

Beispiel B14: Herstellung von

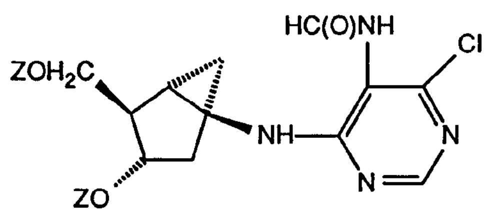

(20).

Z = Butyldimethylsilyl-

Zu einer Lösung von 2,16 g (5,82 mmol) Verbindung (7) in 50 ml absolutem Dioxan gibt man 2,42 ml (17,46 mmol) absolutes $N(C_2H_5)_3$, 1,22 g (6,40 mmol) 5-Formamido-4,6-dichlorpyrimidin sowie 1 Spatelspitze N,N-Dimethyl-4-aminopyridin. Es wird 18 h bei 50°C gerührt, die gelbliche Suspension mit Diethylether verdünnt, filtriert und das Filtrat eingedampft. Die Flash-Chromatographie an 180 g Kieselgel [$CH_2Cl_2$/Essigsäureethylester/$N(C_2H_5)_3$ (4/1/0,05)] sowie erneute Chromatographie der erhaltenen Mischfraktionen an 80 g Kieselgel mit demselben Elutionsmittel ergeben insgesamt 2,62 g (85 %) der Titelverbindung als praktisch weissen Schaum.

$^{1}$H-NMR (250 MHz, $CDCl_3$) (2 Rotationsisomere): 8,30 (s, Hauptisomer), 8,24 (s, Nebenisomer), 8,20 (s, Hauptisomer), 7,97 (d, breit, Nebenisomer), [2H, C(2)H + CHO]; 7,00 (s, breit, Hauptisomer), 6,67 (d, breit, Nebenisomer); [1H, NH-CHO]; 5,80 [s, breit, NH]; 4,13 [d, J = 5,5 Hz, 1H, C(3')H]; 3,78 [dd, J = 18 Hz, J = 9 Hz, 1H, C(5')H]; 3,74 [dd, breit, 1H, C(5')H]; 3,63 [dd, J = 10 Hz, J = 5,5 Hz, 1H, C(5')H]; 2,45 [m, breit, 1H, C(2')H-β]; 1,98 [m, C(4')H]; 1,82 [d, J = 12 Hz, 1H, C(2')H-α]; 1,40-1,12 [m, C(6')H + C(7')H-endo]; 0,82, 0,79 [2 überlappende s, $CH_3$ (tert-Butyl) überlagern Resonanz für C(7')H-exo]; 0,00 [s, 6H, 2 x Si-$CH_3$]; -0,05 [2 überlappende s, 6H, Si-$CH_3$].

Beispiel B15: Herstellung von

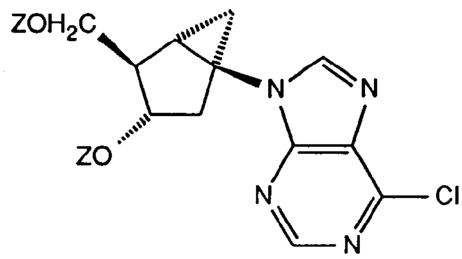

(21).

Z = t-Butyldimethylsilyl-

Eine Lösung von 1,25 g (2,38 mmol) Verbindung (20) in 12,5 ml Diethoxymethylacetat wird 18 h auf 130°C erhitzt. Das Lösungsmittel wird dann am Rotationsverdampfer entfernt und der verbleibende Rückstand für 30 min mit einem Gemisch aus 48 ml Methanol und 4 ml konzentriertem wässrigen Ammoniak behandelt. Der nach dem Eindampfen dieser Mischung erhaltene Rückstand wird in 100 ml Diethylether gelöst, die Lösung 2x mit je 50 ml gesättigter $NaHCO_3$-Lösung extrahiert, getrocknet und eingedampft und der verbleibende Rückstand an 80 g Kieselgel mit $CH_2Cl_2$/Essigsäureethylester/$N(C_2H_5)_3$ (4/1/0,05) chromatographiert. Nach erneuter Chromatographie an 180 g Kieselgel im gleichen Elutionssystem erhält man 750 mg Verbindung (21) (62 %) als amorphen Festkörper.

$^1$H-NMR (250 MHz, $CDCl_3$): 8,68 [s, 1H, C(2)H]; 8,15 [s, 1H, C(8)H]; 4,30 [d, J = 5,5 Hz, 1H, C(3')H]; 3,88 [m, 2H, C(5')$H_2$]; 2,52 [ddd J = 14,0 Hz, J = 5,5 Hz, J < 1 Hz, 1H, C(2')H-β]; 2,19 [d, J = 14,0 Hz, überlappend mit m, 2H, C(2')H-α + C(4')H]; 1,75 [m, 2H, C(6')H + C(7')H-endo]; 1,33 [m, 1H, C(7')H-exo]; 0,89 [s, 9H, $CH_3$ (tert-Butyl)]; 0,85 [s, 9H, $CH_3$ (tert-Butyl)]; 0,08 [2 überlappende s, 2 x Si-$CH_3$; 0,00 [2 überlappende s, 2 x Si-$CH_3$].

Beispiel B16: Herstellung von

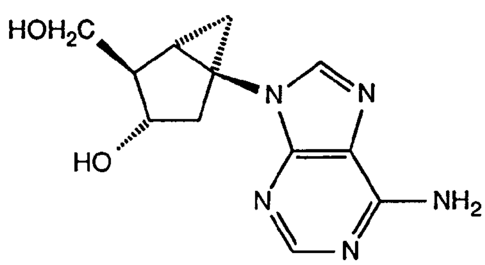

(22).

In eine Lösung von 1,69 g (3,31 mmol) Verbindung (21) in 20 ml Methanol wird bei -78°C in einer Fischer-Porter Apparatur solange gasförmiger Ammoniak einkondensiert bis sich das Volumen der Mischung etwa verdoppelt hat. Das Rohr wird dann verschlossen, auf RT erwärmt und die Reaktionsmischung für 20 h bei 60°C gerührt. Das Gefäss wird bei 0°C geöffnet, der Ammoniak bei RT und Normaldruck abgedampft und die verbleibende Lösung am Rotationsverdampfer eingedampft. Der verbleibende feste Rückstand wird in 20 ml absolutem THF gelöst, 20 ml einer 1M Lösung von Tetrabutylammoniumfluorid in THF zugegeben und dann 3 h bei 60°C gerührt. Es werden 20 g Ionenaustauscher (Levatit S 108061/$H^+$-Form), 20 ml Methanol und 1 ml Wasser zugegeben und das Gemisch 2 h bei 60°C gerührt. Der Ionenaustauscher wird abfiltriert, dreimal mit MeOH gewaschen und anschliessend in 50 ml Methanol suspendiert. Die Mischung wird durch Zugabe von konzentriertem wässrigem Ammoniak alkalisch gestellt, kurz zum Rückfluss erhitzt und der Ionenaustauscher heiss abfiltriert. Es wird anschliessend noch zweimal in heissem Methanol aufgeschlämmt und filtriert. Die vereinigten Filtrate werden am Rotationsverdampfer eingedampft und der feste Rückstand in 10 ml Ethanol suspendiert. Die Filtration ergibt 671 mg (78 %) der Titelverbindung als weisse Kristalle.

$^1$H-NMR (500 MHz, DMSO-$d_6$): 8,136, 8,126 [2 x s, C(2)H + C(8)H]; 7,24 [s, breit, 2H, $NH_2$]; 5,22 [dd, J = 7,0 Hz, J = 4,5 Hz, 1H, $CH_2OH$]; 4,76 [d, 1H, J = 3 Hz, 1H, CHOH]; 4,16 [d, breit, J = 6 Hz, 1H, C(3')H]; 3,70 [ddd (quintettoid), J = 11 Hz, J = 6,5 Hz, J = 6,5 Hz, 1H, C(5')H]; 2,42 [ddd, J = 13,0 Hz, J = 6,5 Hz, J = 1,5 Hz, 1H, C(2')H-β]; 2,11 [d, J = 13,0 Hz, 1H, C(2')H-α]; 2,02 [dd (triplettoid), J = 5,5 Hz, J = 5,5 Hz, 1H, C(4')H]; 1,73 [ddd, J = 9 Hz, J = 4,5 Hz, J ~ Hz, 1H, C(6')H]; 1,53 [dd (triplettoid), J = 4,5 Hz, J = 4,5 Hz, 1H, C(7')H-endo]; 1,30 [ddd, J = 9 Hz, J = 4,5 Hz, J ~ 2 Hz, 1H, C(7')H-exo].

Beispiel B17: Herstellung von

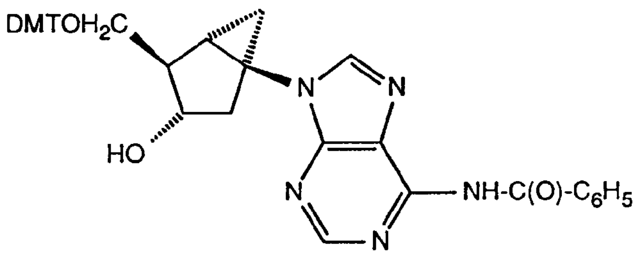

(23).

DMT = 4,4'-Dimethoxytrityl

Zu einer Suspension von 392 mg (1,5 mmol) Verbindung (22) in 14 ml absolutem Pyridin gibt man bei 0°C 951 µl (7,5 mmol) Trimethylchlorsilan. Nach 30 min Rühren bei 0°C tropft man ebenfalls bei 0°C 871 µl (7,5 mmol) Benzoylchlorid zu, lässt nach Entfernung des Kältebades auf RT aufwärmen, rührt noch 2 h bei RT und lässt dann bei 0°C innerhalb von 5 min 3 ml Eiswasser zutropfen. Nach 15 min bei 0°C tropft man 3 ml konzentrierten wässrigen Ammoniak zu, rührt 30 min bei RT und dampft dann am Rotationsverdampfer ein. Der so erhaltene feste Rückstand wird in 50 ml Wasser gelöst, mit 50 ml Diethylether ausgeschüttelt und die wässrige Phase erneut eingedampft. Die Reinigung an 80 g Kieselgel mit Essigsäureethylester/Methanol als Laufmittel ergibt das $N^6$-Benzoyl-Derivat von Verbindung (22), das noch mit Benzamid verunreinigt ist und als solches in die nächste Stufe eingesetzt wird.

$^1$H-NMR (250 MHz, $CD_3OD$): u.a. 8,40, 8,18 [2 x s, 2 x 1H, C(2)H + C(8)H]; 7,35-7,05 [m, aromat. H]; 4,10 [d, J = 6,5 Hz, 1H, C(3')H]; 3,69 [dd, J = 11,0 Hz, J = 5,0 Hz, 1H, C(5')H]; 3,59 [dd, J = 11,0 Hz, J = 5,5 Hz, 1H, C(5')H]; 2,30 [m, 1H, C(2')H-β]; 2,01 [d, J = 13,5 Hz, 1H, C(2')H-α]; 1,92 [dd (triplettoid), J = 5,0 Hz, J = 5,0 Hz, 1H, C(4')H]; 1,62 [m, 1H, C(6')H]; 1,42 [dd (triplettoid), J = 6,0 Hz, J = 6,0 Hz, 1H, C(7')H-endo]; 1,19 [m, 1H, C(7')H-exo].

Das erhaltene Rohprodukt wird in 20 ml absolutem Pyridin gelöst, 291 µl (2,1 mmol) $N(C_2H_5)_3$, 610 mg (1,8 mmol) 4,4-Dimethoxytritylchlorid sowie eine Spatelspitze N,N-Dimethyl-4-aminopyridin zugegeben und die Lösung wird 18 h bei RT gerührt. Das Gemisch wird dann auf 50 ml Wasser und 50 ml Diethylether gegossen, die organische Phase abgetrennt und die wässrige Phase noch dreimal mit je 50 ml Diethylether extrahiert. Die vereinigten organischen Extrakte werden über $MgSO_4$ getrocknet, eingedampft und der Rückstand an 80 g Kieselgel mit Essigsäureethylester und wenig $N(C_2H_5)_3$ flashchromatographiert. Es werden 532 mg (53 % bezogen auf (22)) der Titelverbindung als weisser Schaum erhalten.

$^1$H-NMR (250 MHz, $CDCl_3$): 9,58 [s, breit, 1H, NH]; 8,59 [s, 1H]; 8,04 [d, J = 7,5 Hz, 2H, H aromat. o zu CONH]; 7,88 [s, 1H]; 7,60-7,18 [m, 12H]; 6,83 [d, J = 7,0 Hz, 4H, H aromat. o zu $OCH_3$]; 4,45 [d, J = 5,5 Hz, C(3')H]; 3,78 [s, breit, 7H, $OCH_3$ + OH]; 3,44 [m, 2H, C(5')$H_2$]; 2,55-2,32 [m, 2H]; 2,22 [d, J = 13,0 Hz, 1H]; 1,90-1,70 [m, 2H], 1,36 [m, 1H].

Beispiel B18: Herstellung von

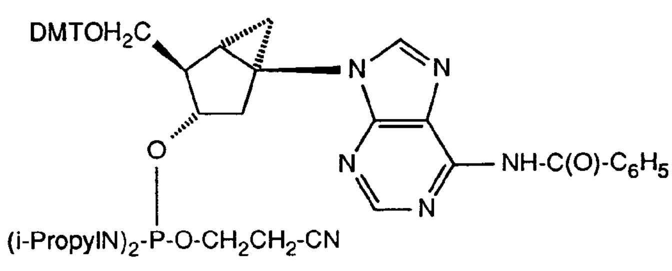

(24).

DMT = 4,4'-Dimethoxytrityl

Analog Beispiel B4 erhält man aus 530 mg (0,797 mmol) Verbindung (23) und 264 mg (0,877 mmol) β-Cyanoethoxy-Bis(diisopropylamino)phosphin 529 mg (79 %) der Titelverbindung (Diastereomerengemisch) als stark elektrostatisch geladenes weisses Pulver.

$^1$H-NMR (250 MHz, $CDCl_3$): 9,25 [s, 1H, NH]; 8,58 [2 überlappende s, 1H, C(2)H]; 8,00 [d, 2H, H aromat o zu CONH]; 7,90 [2 überlappende s, 1H, C(8)H]; 7,60-7,15 [m, H aromat.]; 6,85 [m, 4H, H aromat. o zu $OCH_3$]; 4,50 [m, 1H, C(3')H]; 3,85-3,40 [m inkl. s bei 3,78 ($OCH_3$), 12H]; 2,65-2,30 [m, 6H (inkl. $CH_3$ von nicht vollständig entferntem Toluol]; 1,82 [m, 1H]; 1,72 [m, 1H]; 1,42 [m, 1H]; 1,35-1,10 [m, $CH_3$ (Isopropyl)].

$^{31}P$-NMR ($CDCl_3$): 148,26; 148,10.

Beispiel B19: Herstellung von

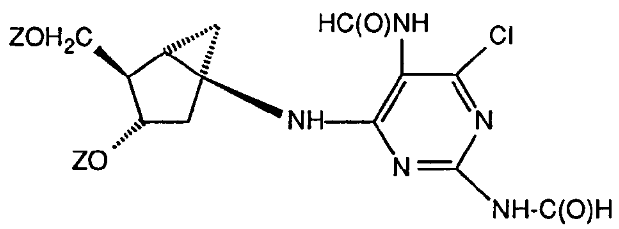

(25).

Z = t-Butyldimethylsilyl-

Zu einer Lösung von 2,25 g (6,05 mmol) von Verbindung (7) in 50 ml Dioxan gibt man 3,35 ml (24,2 mmol) absolutes $N(C_2H_5)_3$ und 1,49 g (6,35 mmol) 2,5-Bis(formamido)-4,6-dichlorpyrimidin. Das Gemisch wird 1,5 h am Rückfluss erhitzt, dann auf 0°C gekühlt, mit 50 ml Diethylether verdünnt, die Lösung filtriert und das Filtrat eingedampft. Der Rückstand wird an 180 g Kieselgel mit Essigsäureethylester flash-chromatographiert. Man erhält so 2,0 g der Titelverbindung als weissen Schaum. Erneute Chromatographie der in der ersten Chromatographie erhaltenen Mischfraktionen liefert weitere 820 mg an Verbindung (25). Gesamtausbeute: 2,82 g (82 %).

$^1H$-NMR (250 MHz, $CD_3OD$): 9,30 [s, breit, 1H, C<u>H</u>O]; 8,12 [s, breit, 1H, C<u>H</u>O]; 4,14 [d, J = 4,0 Hz, 1H, C(3')<u>H</u>]; 3,63 [dd, J = 9,5 Hz, J = 6,0 Hz, 1H, C(5')<u>H</u>]; 3,46 [dd (triplettoid), J = 9,5 Hz, J = 9,5 Hz, 1H, C(5')<u>H</u>]; 2,12 [m, 1H, C(2')<u>H-β</u>]; 2,06 [d, J = 12,0 Hz, 1H, C(2')<u>H-α</u>]; 1,93 [dd, J = 9,5 Hz, J = 4,0 Hz, 1H, C(4')<u>H</u>]; 1,36 [m, 2H, C(6')<u>H</u> + C(7')H-<u>endo</u>]; 0,83 [2 überlappende s, C<u>H</u>$_3$ (tert-Butyl), + C(7')<u>H-exo</u>]; 0,00 [2 überlappende s, Si-C<u>H</u>$_3$].

Beispiel B20: Herstellung von

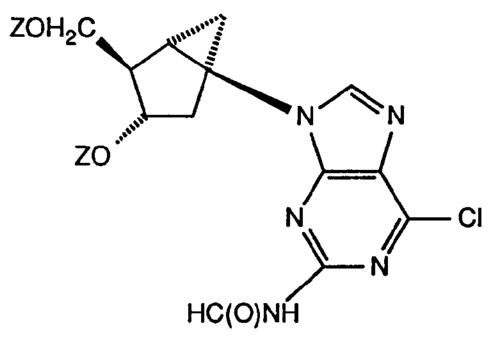

(26).

Z = t-Butyldimethylsilyl-

Eine Lösung von 2,80 g (4,91 mmol) von Verbindung (25) in 25 ml Diethoxymethylacetat wird 19 h bei 140°C gerührt. Das Lösungsmittel wird dann am Rotationsverdampfer ent-fernt, der Rückstand in 18 ml Methanol gelöst, 1,5 ml konzentrierter wässriger Ammoniak zugegeben und 30 min bei RT gerührt. Das Gemisch wird dann am Rotationsverdampfer eingedampft, der Rückstand in 100 ml Essigsäureethylester gelöst, zweimal mit 50 ml $H_2O$ ausgeschüttelt, die organische Phase getrocknet und eingedampft. Der Rückstand wird an 180 g Kieselgel mit $CH_2Cl_2$/Essigsäureethylester/$N(C_2H_5)_3$ (9/1/0,1) flash-chro-matographiert. Es werden 1,32 g der Titelverbindung als amorpher Festkörper erhalten. Erneute Chromatographie der bei der ersten Chromatographie erhaltenen produkthaltigen Mischfraktionen liefert weitere 270 mg an (26), was einer Gesamtausbeute von 59 % entspricht.

$^1H$-NMR (250 MHz, $CDCl_3$): 9,38 [d, J = 11,5 Hz, 1H, C<u>H</u>O]; 8,30 [d, J = 11,5 Hz, 1H, N<u>H</u>]; 7,97 [s, 1H, C(8)<u>H</u>]; 4,18 [d, J = 5,0 Hz, 1H, C(3')<u>H</u>]; 3,72 [m, 2H, C(5')<u>H</u>]; 2,38 [ddd, J = 13 Hz, J = 6,0 Hz, J ~ 1 Hz, 1H, C(2')<u>H-β</u>]; 2,15-2,00 [m, 2H, C(2')<u>H-α</u> + C(4')<u>H</u>]; 1,73-1,60 [m, 2H, C(6')<u>H</u> + C(7')<u>H-endo</u>]; 1,18 [m, 1H, C(7')<u>H-exo</u>]; 0,79, 0,77 [2 überlappende s, C<u>H</u>$_3$ (tert-Butyl)]; 0,00 [s, 6H, Si-C<u>H</u>$_3$]; -0,08 [2 überlappende s, 6H, Si-C<u>H</u>$_3$].

Beispiel B21: Herstellung von

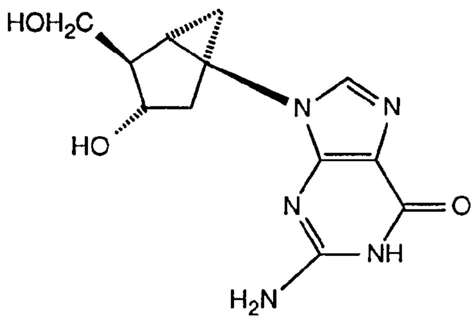

(27).

Eine Lösung von 1,59 g (2,88 mmol) Verbindung (26) in 20 ml 80%iger Ameisensäure wird 1 h am Rückfluss erhitzt. Das Lösungsmittel wird dann am Rotationsverdampfer entfernt, der Rückstand mit 20 ml konzentriertem wässrigen Ammoniak und 10 ml Methanol versetzt und das Gemisch 1 h bei 40°C gerührt. Die Lösung wird eingedampft und der Rückstand über 120 g Kieselgel mit Methanol als Elutionsmittel chromatographiert. Der so erhaltene Festkörper wird in 50 ml Methanol gelöst, 3 ml $N(C_2H_5)_3$ zugegeben und 30 min bei RT gerührt. Der entstandene Niederschlag wird abfiltriert, in 10 ml wässrigem Isopropanol (50 %) suspendiert, erneut filtriert, mit Isopropanol und Diethylether gewaschen und im Hochvakuum getrocknet. Man erhält 705 mg (88 %) der Titelverbindung als weisses Pulver.

$^1$H-NMR (500 MHz, DMSO): 8,49 [s, 1H, NH]; 7,62 [s, 1H, C(8)H]; 6,50 [s, breit, 2H, $NH_2$]; 4,90 [s, breit, 1H, OH]; 4,71 [s, breit, 1H, OH]; 4,09 [d, J = 5,5 Hz, 1H, C(3')H]; 3,58 [d, J = 4,5 Hz, 2H, C(5')$H_2$]; 2,24 [ddd, J = 10,5 Hz, J = 5,5 Hz, J = 1 Hz, 1H, C(2')H-β]; 2,09 [d, J = 10,5 Hz, 1H, C(2')H-α]; 1,96 [dd (triplettoid), J = 4,5 Hz, J = 4,5 Hz, 1H, C(4')H]; 1,71 [dd, J = 7 Hz, J = 3,5 Hz, 1H, C(6')H]; 1,46 [dd (triplettoid); J = 3,5 Hz, J = 3,5 Hz, 1H, C(7')H-endo]; 1,14 [ddd, J = 7 Hz, J = 3,5 Hz, J = 1,2 Hz, 1H, C(7')H-exo].

Beispiel B22: Herstellung von

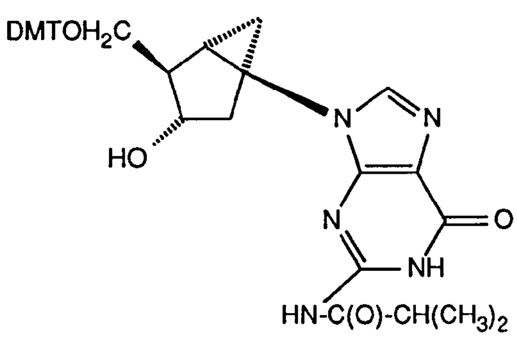

(28).

DMT = 4,4'-Dimethoxytrityl

Zu einer Suspension von 616 mg (2,22 mmol) Verbindung (27) in 20 ml absolutem Pyridin tropft man bei 0°C 1,41 ml (11,1 mmol) Trimethylchlorsilan. Nach 10 min bei 0°C wird eine Spatelspitze N,N-Dimethyl-4-aminopyridin zugegeben und die Mischung auf RT erwärmt. Nach 30 min bei RT (wobei Lösung eintritt) wird wieder auf 0°C gekühlt, 1,89 ml Isobuttersäureanhydrid zugegeben und dann 2 h bei RT gerührt. Es werden 4,4 ml Eiswasser zugegeben und nach 15 min bei 0°C und Zugabe von 4,4 ml konzentriertem wässrigen Ammoniak wird 1 h bei RT gerührt. Der nach Abdampfen des Lösungsmittels erhaltene Rückstand wird an 150 g Kieselgel mit einem Gradienten von Essigsäureethylester/Methanol 9/1 bis 100 % MeOH chromatographiert, der so erhaltene Festkörper für 2,5 h bei 30-35°C mit 30 ml Methanol/konzentriertem Ammoniak (2/1) behandelt, die Lösung eingedampft und der Rückstand erneut mit Essigsäureethylester/Methanol (2/1) chromatographiert. Auf diese Weise erhält man 560 mg des $N^2$-Isobutyryl-Derivats von Verbindung (27), das mit Isobuttersäureamid verunreinigt ist und als solches in der nächsten Stufe eingesetzt wird.

$^1$H-NMR (250 MHz, $CD_3OD$): u. a. 7,90 [s, 1H, C(8)H]; 4,14 [d, J = 6,0 Hz, 1H, C(3')H]; 3,70 [m, 2H, C(5')$H_2$]; 2,68 [m, 1H, $CH(CH_3)_2$]; 2,18 [d, J = 14,0 Hz, 1H]; 2,06 [dd (triplettoid), J = 5,5 Hz, J = 5,5 Hz, 1H]; 1,90 [m, 1H]; 1,51 [dd (triplettoid), J = 4,5 Hz, J = 4,5 Hz, 1H]; 1,28 [m, 1H]; 1,13 [d, J = 6,0 Hz, $CH(CH_3)_2$].

Zu einer Lösung von 560 mg des Rohprodukts in 30 ml absolutem Pyridin gibt man 312 µl (2,25 mmol) absolutes $N(C_2H_5)_3$, 654 mg (1,93 mmol) 4,4'-Dimethoxytritylchlorid sowie eine Spatelspitze N,N-Dimethyl-4-aminopyridin und lässt bei RT rühren. Nach 2 h gibt man weitere 130 mg 4,4'-Dimethoxytritylchlorid und 62 µl $N(C_2H_5)_3$ zu. Nach insgesamt 18 h wird die Reaktionsmischung auf 100 ml Eiswasser und 100 ml Diethylether gegossen, die organische Phase abgetrennt, die wässrige Phase noch dreimal mit je 50 ml Diethylether extrahiert, die vereinigten organischen Extrakte über $MgSO_4$ getrocknet und am Rotationsverdampfer eingedampft. Nach Flash-Chromatographie des Rückstands an 180 g Kieselgel mit Essigsäureethylester/Isopropanol/$N(C_2H_5)_3$ (19/1/0,02) als Elutionsmittel erhält man 746 mg (52 % bezogen auf (27) der Titelverbindung als blass gelblich gefärbten Schaum.

$^1$H-NMR (250 MHz, $CD_3OD$): u.a. 7,42 [s, 1H, C(8)H]; 7,38-6,95 [m, 9H]; 6,70-6,60 [m, 4H, H aromat. o zu $OCH_3$]; 4,08 [d, J = 4,5 Hz, 1H, C(3')H]; 3,55 [s, 6H, $OCH_3$]; 2,55 [Sextett, 1H, $CH(CH_3)_2$]; 2,28-2,12 [m, 3H]; 1,95 [m, 1H]; 1,48 [dd (triplettoid), 1H]; 1,20 [m, 1H]; 1,07 [d, J = 5,5 Hz, $CH(CH_3)_2$].

Beispiel B23: Herstellung von

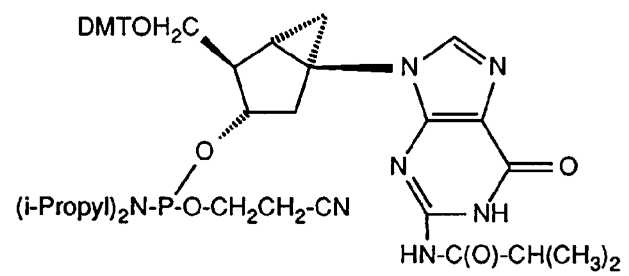

(29).

DMT = 4,4'-Dimethoxytrityl

Analog zu Beispiel B4 erhält man aus 740 mg (1,14 mmol) von Verbindung (28) und 756 mg (2,51 mmol) β-Cyanoethoxy-bis(diisopropylamino)phosphin nach dreimaligem Umfallen 641 mg (64 %) der Titelverbindung (Diastereomerengemisch) als amorphes weisses Pulver.

$^1$H-NMR (250 MHz, $CDCl_3$): 11,97 [s, sehr breit, 1H, NH]; 8,87 [s, breit, ~ 0,5H, NH], 7,98 [s, breit, ~ 0,5H, NH]; 7,55-7,25 [m, 10H]; 6,90-6,75 [m, 4H, H aromat. o zu $OCH_3$]; 4,43 [m, breit, 1H, C(3')H]; 3,85-3,40 [m inkl. s bei 3,80 ($OCH_3$), 10H]; 3,30-3,19 [m, 2H] 2,75-2,50 [m, 2H]; 2,45-2,20 [m, 3H]; 2,12-1,95 [m, 2H] 1,69, 1,52 [2 x "t", 1H]; 1,40-0,95 [m, 19H].

$^{31}$P-NMR ($CDCl_3$): 148,80, 147,36.

## C) Herstellung von Oligonukleotiden

### Beispiele C1-C4:

Oligonukleotide werden unter Verwendung der erfindungsgemässen dimethoxytritylierten und 3'-aktivierten [3'-(β-Cyanoethoxy-di(i-propylamino)phosphoramidit)] Nukleoside bzw. solcher natürlicher aktivierten Nukleosiden an einen festen Träger gebunden (Controlled Pore Glas, CPG) und die Synthese auf einem DNA-Synthesiser (Applied Biosystems, Modell 380 B, Standard Phosphoramiditchemie und Iodoxidation) gemäss den Standardprotokollen des Herstellers durchgeführt [vergleiche auch "Oligonucleotide synthesis, a practical approach" M.J Gait; IRL Press 1984 (Oxford-Washington DC)]. Nach der Kopplung des letzten Nukleosidbausteins wird das 5'-geschützte Oligonukleotid unter gleichzeitiger Abspaltung aller übrigen Schutzgruppen durch Behandlung mit konzentriertem wässrigen Ammoniak über Nacht vom Träger abgelöst und anschliessend unter Verwendung von 50 mM Ammoniumacetatpuffer (pH 7)/Acetonitril durch "reverse-phase"-HPLC gereinigt. Anschliessend wird die 5'-Dimethoxytrityl-Schutzgruppe durch 20-minütige Behandlung mit 80 %-iger wässriger Essigsäure abgespalten, das Oligonukleotid mit Ethanol ausgefallt und durch Zentrifugation isoliert. Die Reinheit des Oligonukleotids wird durch Gelelektrophorese (Polyacrylamid) überprüft.

### Beispiel C1:

Aus der Verbindung von Beispiel B2 (t) bzw. B4 und den O-5'-Dimethoxytrityl-geschützten O-3'-β-Cyanoethoxy-N,N-diisopropylaminophosphoramiditen der natürlichen Nukleoside 2'-Desoxyadenosin (dA), 2'-Desoxycytidin (dC) und 2'-Desoxyguanosin (dG) wird ein Oligonukleotid mit der Sequenz d(tCC AGG tGt CCG CAt C) hergestellt.

Beispiel C2:

Aus der Verbindung von Beispiel B2 (t) bzw. B4 und den natürlichen Nukleosiden dC, dA, dG und Thymidin (dT) wird ein Oligonukleotid mit der Sequenz d(CTC GTA CCt TTC CGG TCC) hergestellt.

Beispiel C3:

Aus der Verbindung von Beispiel B2 (t) bzw. B4 und den natürlichen Nukleosiden dA, dC, dG und dT wird ein Oligonukleotid mit der Sequenz d(TCC AGG TGT CCG ttt C) hergestellt.

Beispiel C4:

Aus der Verbindung von Beispiel B2 (t) bzw. B4 und den natürlichen Nukleosiden dC, dA, dG und dT wird ein Oligonukleotid mit der Sequenz d(CTC GTA Ctt ttC CGG TCC) hergestellt.

D) Anwendungsbeispiele

Beispiel D1:

Wechselwirkung der Oligonukleotide gemäss den Beispielen C1 bis C4 mit komplementären Oligonukleotidsequenzen.
Die Wechselwirkung der Oligonukleotide gemäss den Beispielen C1 bis C4 mit den entsprechenden basenkomplementären Oligomeren der natürlichen Desoxy- und Ribonukleotide werden durch das Aufzeichnen von UV-Schmelzkurven und den daraus ermittelten $T_m$-Werten charakterisiert. Diese Standardmethode ist zum Beispiel von L.A. Marky et al. in Biopolymers, 26:1601 ff(1987) beschrieben.
Es wird eine Lösung der Oligonukleotide gemäss den Beispielen C1 bis C4 und der entsprechenden basenkomplementären natürlichen Oligodesoxy- bzw. Oligoribonukleotide in 10 mM Phosphatpuffer, 100 mM NaCl, 0,1 mM EDTA, pH = 7,0 (c = $4 \cdot 10^{-6}$ M/Oligonukleotid) hergestellt und die Änderung der Extinktion bei 260 nm in Abhängigkeit von der Temperatur (15 bis 95 °C) aufgezeichnet. Aus den erhaltenen Schmelzkurven wird der $T_m$-Wert ermittelt (siehe Tabelle 1).

Tabelle 1:

| Oligomer | Oligonukleotid | $T_m$ Wert (°C) | $T_m$ Wert (°C)$^{a)}$ |
|---|---|---|---|
| C1 | RNA | 55.1 | 62.3 |
| C1 | DNA | 53.5 | 62.5 |
| C2 | RNA | 62.4 | 63.3 |
| C2 | DNA | 58.8 | 61.7 |
| C3 | RNA | 56.3 | 59.2 |
| C3 | DNA | 53.1 | 59.0 |
| C4 | RNA | 53.4 | 56.7 |
| C4 | DNA | 49.5 | 58.1 |

$^{a)}$ $T_m$ Werte der unmodifizierten (natürlichen) Analogen
RNA = komplementäres Oligoribonukleotid
DNA = Komplementäres Oligodesoxyribonukleotid

Beispiel D2:

Wechselwirkung des Oligonukleotids gemäss Beispiel C2 mit basenkomplementären Oligonukleotiden
Man stellt Lösungen des Oligonukleotids gemäss Beispiel C2 mit den entsprechenden basenkomplementären

Oligonukleotiden der Sequenzen d(GGA CCG GAA YGG TAC GAG) und r(GGA CCG GAA YGG TAC GAG) in 10 mM Phosphatpuffer, 100 mM NaCl, 0,1 mM EDTA, pH 7, ($c = 4\cdot10^{-6}$ M/Oligonukleotid) her und misst die Änderung der Extinktion bei 260 nm in Abhängigkeit von der Temperatur (15 °C bis 95 °C). Aus den Kurven wird der $T_m$-Wert ermittelt. Die Ergebnisse sind in der Tabelle 2 angegeben.

Tabelle 2:

| Y | Oligonukleotid | $T_m$ Wert (°C) | $T_m$ Wert (°C)[a)] |
|---|---|---|---|
| rA | RNA | 62.4 | 63.3 |
| dA | DNA | 58.8 | 61.7 |
| rC | RNA | 51.6 | 54.4 |
| dC | DNA | 43.1 | 46.9 |
| rG | RNA | 58.2 | 61.7 |
| dG | DNA | 53.1 | 54.8 |
| rU | RNA | 52.0 | 55.9 |
| dU | DNA | 53.5 | 55.7 |

[a)] $T_m$-Werte der unmodifizierten (natürlichen) Analogen
RNA = Oligoribonukleotid r(GGA CCG GAA YGG TAC GAG)
DNA = Oligodesoxyribonukleotid d(GGA CCG GAA YGG TAC GAG)

Beispiel D3:

Enzymatische Hydrolyse des Oligonukleotids gemäss Beispiel C3. Je 14 µg des Oligonukleotids gemäss Beispiel C3 beziehungsweise des entsprechenden natürlichen Oligomers werden in 200 µl 10%-igem Hitze inaktivierten Serum aus Kalbsföten bei 37 °C inkubiert (c = 70 µg/ml). Nach 0,5; 1; 2; 4; 6 und 21 Stunden werden jeweils 15 µl der Reaktionslösung durch Zugabe zu 25 µl 9M Harnstoff und Trisborat-Puffer (pH 7) gequencht und bis zur Messung bei -20 °C gelagert. Die gequenchten Reaktionslösungen werden mittels Polyacrylamid Gelelektophorese aufgetrennt und die Spaltprodukte über den Phosphorgehalt bestimmt (Phospho-imagers Methode). Das Verhältnis R zwischen der Summe der Konzentration des völlig intakten Oligonukleotids ($c_n^{(t)}$) und der Konzentration des durch Abspaltung des natürlichen C-Bausteins vom 3'-Ende entstehenden Fragments ($c_{n-1}^{(t)}$) zu einer gegebenen Zeit t und der Ausgangskonzentration des völlig intakten Oligonukleotids zur Zeit t = 0 ($c_n^{(0)}$) [$R = c_n^{(t)} + c_{n-1}^{(t)}/c_n^{(0)}$ beträgt bei Verwendung des Oligomers gemäss Beispiel C3 1 (4 h), 0,98 (6 h) und 0,92 (21 h), bei Verwendung des entsprechenden natürlichen Oligomers mit der Sequenz d(TTC AGG TGT CCG TTT C) 0,11 (0,5 h), 0,05 (1 h) und 0,01 (2 h).

**Patentansprüche**

1. Verbindung der Formeln I und Ia und ihre Racemate

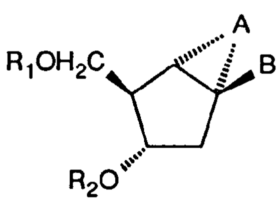 (I), 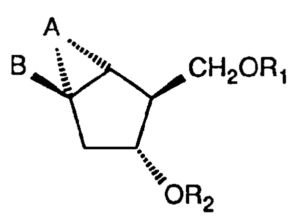 (Ia),

worin A für $-CH_2-$ oder $-CH_2CH_2-$ steht, $R_1$ für Wasserstoff oder eine Schutzgruppe steht, $R_2$ für Wasserstoff oder eine Schutzgruppe oder einen eine phosphorhaltige Nukleotid-Brückengruppe bildenden Rest steht und B einen Purin- oder Pyrimidinrest oder ein Analoges davon bedeutet.

2. Verbindung gemäss Anspruch 1, dadurch gekennzeichnet, dass R1 und $R_2$ je für Wasserstoff stehen.

3. Verbindung gemäss Anspruch 1, dadurch gekennzeichnet, dass A für $-CH_2-$ steht.

4. Verbindung gemäss Anspruch 1, dadurch gekennzeichnet, dass $R_1$ und $R_2$ unabhängig voneinander lineares oder verzweigtes $C_1$-$C_8$-Alkyl, $C_7$-$C_{18}$-Aralkyl, Triphenylsilyl, Alkyldiphenylsilyl, Dialkylphenylsilyl oder Trialkylsilyl mit 1 bis 20 C-Atomen in den Alkylgruppen, $-(C_1$-$C_8$-Alkyl$)_2$Si-O-Si($C_1$-$C_8$-Alkyl$)_2$-. $C_2$-$C_{12}$-Acyl, $R_3$-$SO_2$-, worin $R_3$ $C_1$-$C_{12}$-Alkyl bedeutet, $C_5$- oder $C_6$-Cycloalkyl, Phenyl, Benzyl, $C_1$-$C_{12}$-Alkylphenyl, $C_1$-$C_{12}$-Alkylbenzyl, Halogenphenyl oder Halogenbenzyl bedeutet, oder unsubstituiertes oder mit F, Cl, Br, $C_1$-$C_4$-Alkoxy, Tri-($C_1$-$C_4$-Alkyl)silyl oder $C_1$-$C_4$-Alkylsulfonyl substituiertes $C_1$-$C_{12}$-Alkoxycarbonyl, Phenyloxycarbonyl, Benzyloxycarbonyl, Methylphenyloxycarbonyl oder Methylbenzyloxycarbonyl oder 9-Fluorenylmethyloxycarbonyl darstellen.

5. Verbindung gemäss Anspruch 4, dadurch gekennzeichnet, dass $R_1$ und $R_2$ unabhängig voneinander lineares oder verzweigtes $C_1$-$C_4$-Alkyl, $C_7$-$C_{18}$-Aralkyl, Trialkylsilyl mit 1 bis 12 C-Atomen in den Alkylgruppen, $-(CH_3)_2Si-O-Si(CH_3)_2-$, $-(i-C_3H_7)_2Si-O-Si(iC_3H_7)_2-$, $C_2$-$C_8$-Acyl, $R_3$-$SO_2$-, worin $R_3$ $C_1$-$C_6$-Alkyl bedeutet, Phenyl, Benzyl, C1-$C_4$-Alkylphenyl, $C_1$-$C_4$-Alkylbenzyl, Halogenphenyl oder Halogenbenzylbedeutet, oder $C_1$-$C_8$-Alkoxycarbonyl, Phenyloxycarbonyl, Benzyloxycarbonyl oder 9-Fluorenylmethyloxycarbonyl darstellen.

6. Verbindung gemäss Anspruch 4, dadurch gekennzeichnet, dass $R_1$ und $R_2$ unabhängig voneinander Methyl, Ethyl, n- oder i-Propyl, n-, i- oder t-Butyl; Benzyl, Methylbenzyl, Dimethylbenzyl, Methoxybenzyl, Dimethoxybenzyl, Brombenzyl; Diphenylmethyl, Di(methylphenyl)methyl, Di(dimethylphenyl)methyl, Di-(methoxyphenyl)methyl, Di(methoxyphenyl)(phenyl)methyl, Trityl, Tri(methylphenyl)methyl, Tri(dimethylphenyl)methyl, Tri(methoxyphenyl)methyl, Tri(dimethoxyphenyl)methyl; Trimethylsilyl, Triethylsilyl, Tri-n-propylsilyl, i-Propyl-dimethylsilyl, t-Butyl-dimethylsilyl, t-Butyldiphenylsilyl, n-Octyl-dimethylsilyl, (1,1,2,2-Tetramethylethyl)-dimethylsilyl, $-(CH_3)_2Si-O-Si(CH_3)_2-$, $-(i-C_3H_7)_2Si-O-Si(iC_3H_7)_2-$; Acetyl, Propanoyl, Butanoyl, Pentanoyl, Hexanoyl, Benzoyl, Methylbenzoyl, Methoxybenzoyl, Chlorbenzoyl oder Brombenzoyl; Methyl-, Ethyl-, Propyl-, Butyl-, Phenyl-, Benzyl-, p-Brom-, p-Methoxy- oder p-Methylphenylsulfonyl; Methoxy-, Ethoxy-, n- oder i-Propoxy- oder n-, i- oder t-Butoxycarbonyl, oder Phenyloxycarbonyl, Benzyloxycarbonyl, Methyl- oder Methoxy- oder Chlorphenyloxycarbonyl oder -benzyloxycarbonyl oder 9-Fluorenylmethyloxy-carbonyl darstellen.

7. Verbindung gemäss Anspruch 1, dadurch gekennzeichnet, dass $R_1$ und $R_2$ gleiche Schutzgruppen bedeuten.

8. Verbindung gemäss Anspruch 1, worin B als Purinrest oder einem Analogen davon einen Rest der Formel II, IIa, IIb, IIc, IId oder IIe darstellt,

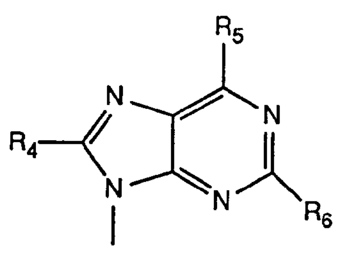

(II),

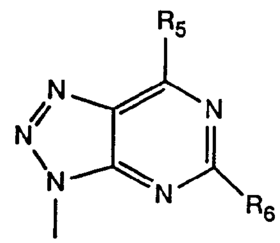

(IIa),

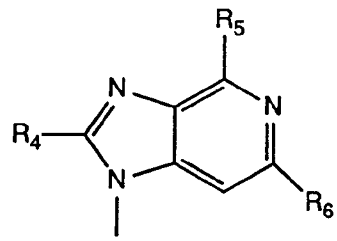

(IIb),

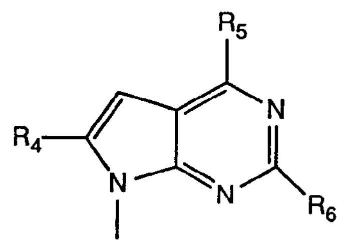

(IIc),

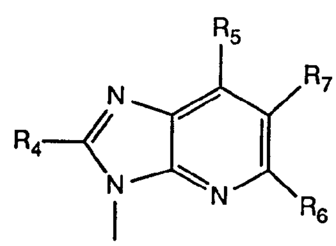

(IId),

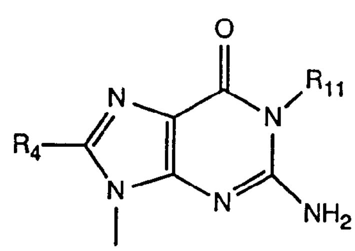

(IIe),

worin $R_4$ für H, Cl, Br oder OH oder -OAkyl mit 1 bis 12 C-Atomen steht, und $R_5$, $R_5$ und $R_7$ unabhängig voneinander H, OH, SH, $NH_2$, $NHNH_2$, NHOH, NHOAlkyl mit 1 bis 12 C-Atomen, -N=CH-N($C_1$-$C_{12}$-Alkyl)$_2$, F, Cl, Br, Alkyl oder Hydroxyalkyl oder Aminoalkyl oder Alkoxy oder Alkylthio mit 1 bis 12 C-Atomen, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten, und $R_{11}$ H oder $C_1$-$C_4$-Alkyl darstellt.

9. Verbindung gemäss Anspruch 8, worin die Schutzgruppe für Hydroxyl- und Aminogruppen $C_1$-$C_8$-Acyl

darstellt.

**10.** Verbindung gemäss Anspruch 8, worin das Primäramino 1 bis 12 C-Atome und das Sekundäramino 2 bis 12 C-Atome enthält.

**11.** Verbindung gemäss Anspruch 8, worin es sich bei dem Primäramino und Sekundäramino um Reste der Formel $R_8R_9N$ handelt, worin $R_8$ für H steht oder unabhängig die Bedeutung von $R_9$ hat, und $R_9$ $C_1$-$C_{20}$-Alkyl, -Aminoalkyl, -Hydroxyalkyl; Carboxyalkyl oder Carbalkoxyalkyl, wobei die Carbalkoxygruppe 2 bis 8 C-Atome und die Alkylgruppe 1 bis 6, bevorzugt 1 bis 4 C-Atome enthält; $C_2$-$C_{20}$-Alkenyl; Phenyl, Mono- oder Di-($C_1$-$C_4$-Alkyl- oder Alkoxy)phenyl, Benzyl, Mono- oder Di-($C_1$-$C_4$-Alkyl- oder -Alkoxy)benzyl; oder 1,2-, 1,3- oder 1,4-Imidazolyl-$C_1$-$C_6$-Alkyl darstellt, oder $R_8$ und $R_9$ zusammen Tetra- oder Pentamethylen, 3-Oxa-1,5-pentylen, $-CH_2-NR_{10}-CH_2CH_2-$ oder $-CH_2CH_2-NR_{10}-CH_2CH_2-$ darstellen, worin $R_{10}$ für H oder $C_1$-$C_4$-Alkyl steht, wobei die Aminogruppe im Aminoalkyl unsubstituiert oder mit ein oder zwei $C_1$-$C_4$-Alkyl oder -Hydroxyalkylgruppen substituiert ist, und die Hydroxylgruppe im Hydroxyalkyl gegebenenfalls mit $C_1$-$C_4$-Alkyl verethert ist.

**12.** Verbindung gemäss Anspruch 10, worin es sich bei dem Primäramino und Sekundäramino um Methyl-, Ethyl-, Dimethyl-, Diethyl-, Allyl-, Mono- oder Di-(hydroxy-eth-2-yl)-, Phenyl-, Benzyl-, Acetyl-, Isobutyryl und Benzoylamino handelt.

**13.** Verbindung gemäss Anspruch 8, worin $R_4$ in den Formeln II, IIb, IIc, IId und IIe für Wasserstoff steht.

**14.** Verbindung gemäss Anspruch 8, worin $R_7$ in Formel IId für Wasserstoff steht.

**15.** Verbindung gemäss Anspruch 8, worin $R_5$ und $R_8$ in den Formeln II, IIa, IIb, IIc, IId und IIe unabhängig voneinander H, F, Cl, Br, OH, SH, $NH_2$, NHOH, $NHNH_2$, Methylamino, Dimethylamino, Benzoylamino, Methoxy, Ethoxy und Methylthio darstellen.

**16.** Verbindung gemäss Anspruch 8, worin B ein Purinrest oder ein Rest eines Purinanalogen aus der Reihe Adenin, N-Methyladenin, N-Benzoyladenin, 2-Methylthioadenin, 2-Aminoadenin, 2-Hydroxypurin, 2-Amino-6-chlorpurin, 2-Amino-6-methylthiopurin, Guanin und N-Isobutyrylguanin ist.

**17.** Verbindung gemäss Anspruch 1, worin B in Formel I als analoger Pyrimidinrest einen Uracil-, Thymin- oder Cytosinrest der Formeln III, IIIa und IIIb darstellt,

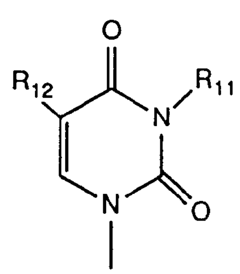

(III),

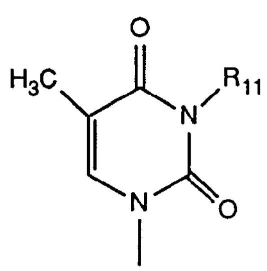

(IIIa)

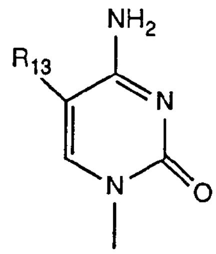

(IIIb),

worin $R_{11}$ H oder $C_1$-$C_4$-Alkyl bedeutet, und $R_{12}$ und $R_{13}$ unabhängig voneinander H, OH, SH, $NH_2$, $NHNH_2$, NHOH, NHOAlkyl mit 1 bis 12 C-Atomen, F, Cl, Br, Alkyl oder Hydroxyalkyl oder Aminoalkyl oder Alkoxy oder Alkylthio mit 1 bis 12 C-Atomen, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten, und die Wasserstoffatome der $NH_2$-Gruppe in Formel IIIb unsubstituiert oder mit $C_1$-$C_6$-Alkyl, Benzoyl oder Benzyl substituiert sind, sowie die Dihydroderivate der Reste der Formeln III, IIIa und IIIb.

**18.** Verbindung gemäss Anspruch 17, worin $R_{12}$ H, $C_1$-$C_6$-Alkyl oder -Hydroxyalkyl, F, Cl, Br, $NH_2$, Benzoylamino, Mono- oder Di-$C_1$-$C_6$-alkylamino darstellt.

**19.** Verbindung gemäss Anspruch 17, worin $R_{13}$ H, $C_1$-$C_6$-Alkyl oder -Alkoxy oder -Hydroxyalkyl, F, Cl, Br, $NH_2$, Benzoylamino, Mono- oder Di-$C_1$-$C_6$-alkylamino darstellt.

**20.** Verbindung gemäss Anspruch 18, worin $R_{12}$ H, F, Cl, Br, $NH_2$, $NHCH_3$, $N(CH_3)_2$ oder $C_1$-$C_4$-Alkyl bedeutet.

**21.** Verbindung gemäss Anspruch 19, worin $R_{13}$ H, $C_1$-$C_4$-Alkyl, $NH_2$, $NHCH_3$ oder $(CH_3)_2N$ bedeutet.

**22.** Verbindung gemäss Anspruch 1, worin B als Rest eines Pyrimidinanalogen sich von Uracil, Thymin, Cytosin, 5-Fluoruracil, 5-Chloruracil, 5-Bromuracil, Dihydrouracil oder 5-Methylcytosin ableitet.

**23.** Verbindung gemäss Anspruch 1, dadurch gekennzeichnet, dass $R_2$ als phosphorhaltiger, eine Nukleotid-Brückengruppe bildender Rest der Formel

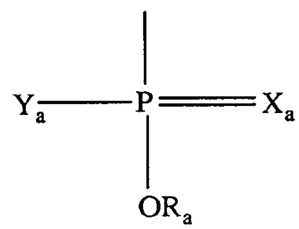

entspricht, worin $Y_a$ Wasserstoff, $C_1$-$C_{12}$-Alkyl, $C_6$-$C_{12}$-Aryl, $C_7$-$C_{20}$-Aralkyl, $C_7$-$C_{20}$-Alkaryl, $-OR_b$, $-SR_b$, $-NH_2$, Primäramino, Sekundäramino, $O^{\ominus}M^{\oplus}$ oder $S^{\ominus}M^{\oplus}$ darstellt; $X_a$ Sauerstoff oder Schwefel bedeutet; $R_a$ Wasserstoff, $M^{\oplus}$, $C_1$-$C_{12}$-Alkyl, $C_2$-$C_{12}$-Alkenyl oder $C_6$-$C_{12}$-Aryl bedeutet, oder die Gruppe $R_aO$- für N-Heteroaryl-N-yl mit 5 Ringgliedern und 1 bis 3 N-Atomen steht; $R_b$ Wasserstoff, $C_1$-$C_{12}$-Alkyl oder $C_6$-$C_{12}$-Aryl bedeutet; und $M^{\oplus}$ für $Na^{\oplus}$, $K^{\oplus}$, $Li^{\oplus}$, $NH_4^{\oplus}$ steht oder Primär-, Sekundär-, Tertiär- oder Quartenärammonium darstellt; wobei Alkyl, Aryl, Aralkyl und Alkaryl in $Y_a$, $R_a$ und $R_b$ unsubstituiert oder mit Alkoxy, Alkylthio, Halogen, -CN, $-NO_2$, Phenyl, Nitrophenyl, oder Halogenphenyl substituiert ist.

**24.** Verbindung gemäss Anspruch 23, dadurch gekennzeichnet, dass $X_a$ für O steht, $R_a$ β-Cyanoethyl bedeutet, und $Y_a$ Di(i-propyl)amino darstellt.

**25.** Verfahren zur Herstellung von Verbindungen der Formel I und/oder Ia gemäss Anspruch 1, dadurch gekennzeichnet, dass man in einer enantiomeren Verbindung der Formel IV oder IVa oder ihren Racematen

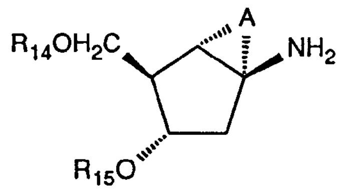
(IV),
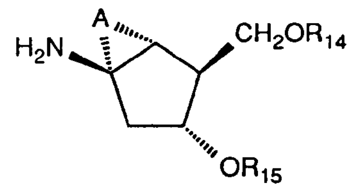
(IVa),

worin A die zuvor angegebene Bedeutung hat und $R_{14}$ und $R_{15}$ für gleiche oder verschiedene Schutzgruppen stehen, in an sich bekannter Weise in einem inerten Lösungsmittel durch Aufbaureaktionen die $NH_2$-Gruppe in einen Rest B umwandelt, wobei B einen Purin- oder Pyrimidinrest oder ein Analoges davon bedeutet.

**26.** Verbindungen der Formeln IV und IVa und deren Racemate

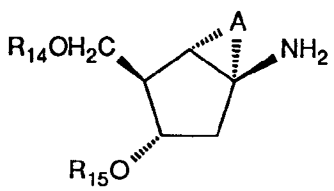
(IV),
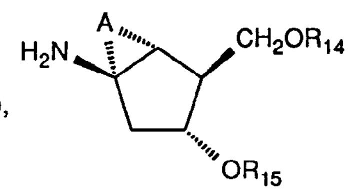
(IVa),

worin A für $-CH_2-$ oder $-CH_2CH_2-$ steht und $R_{14}$ und $R_{15}$ unabhängig H oder gleiche oder verschiedene Schutzgruppen bedeuten.

**27.** Verwendung einer Verbindung der Formeln I und Ia gemäss Anspruch 1 oder deren Racemate zur Herstellung von Oligonukleotiden, die gleiche oder verschiedene Monomereinheiten von Verbindungen der Formeln I und/oder Ia, oder die mindestens eine Monomereinheit von Verbindungen der Formeln I oder Ia und mindestens eine Monomereinheit von anderen natürlichen oder synthetischen Nukleosiden enthalten, wobei die Oligonukleotide 2 bis 200 Monomereinheiten enthalten.

**28.** Verwendung gemäss Anspruch 27 zur Herstellung von Oligonukleotiden mit 2 bis 100 Monomereinheiten.

**29.** Verwendung gemäss Anspruch 28 zur Herstellung von Oligonukleotiden mit 2 bis 50 Monomereinheiten.

**30.** Verwendung gemäss Anspruch 29 zur Herstellung von Oligonukleotiden mit 2 bis 20 Monomereinheiten.

**31.** Verwendung gemäss Anspruch 27 zur Herstellung von Oligonukleotiden mit gleichen oder verschiedenen Monomereinheiten von Verbindungen der Formeln I und/oder Ia.

**32.** Verwendung gemäss Anspruch 27 zur Herstellung von Oligonukleotiden mit gleichen Monomereinheiten von Verbindungen der Formeln I oder Ia und Monomereinheiten von natürlichen oder synthetischen Nukleosiden.

**33.** Oligonukleotid der Formel V

$$5'\text{-U-(O-Y-O-V-)}_y\text{O-Y-O-W-3'} \qquad (V),$$

worin U, V und W je für sich gleiche oder verschiedene Reste von natürlichen oder synthetischen Nukleosiden darstellen und mindestens einer der Reste U, V und/oder W einen Rest der Formeln VI und/oder VIa

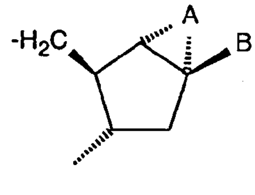
(VI),
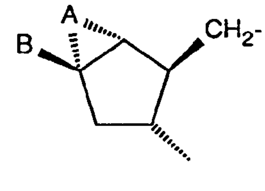
(VIa)

darstellen, und y für eine Zahl von 0 bis 200 steht, Y eine Nukleotid-Brückengruppe darstellt, B einen Purin-

oder Pyrimidinrest oder ein Analoges davon bedeutet, und A für $-CH_2-$ oder $-CH_2CH_2$ steht.

**34.** Oligonukleotid der Formel V gemäss Anspruch 33, dadurch gekennzeichnet, dass es sich bei der Brückengruppe Y um $P(O)O^{\ominus}-$, $-P(O)S^{\ominus}-$, $-P(S)S^{\ominus}-$, $-P(O)R_{16}-$, $-P(O)NR_{17}R_{18}-$, oder $-CH_2-$ handelt, worin $R_{16}$ H oder $C_1$-$C_6$-Alkyl darstellt, und $R_{17}$ und $R_{18}$ unabhängig voneinander die Bedeutung von $R_{16}$ haben.

**35.** Oligonukleotid der Formel V gemäss Anspruch 34, dadurch gekennzeichnet, dass es sich bei der Brückengruppe Y um $-P(O)O^{\ominus}-$ handelt.

**36.** Oligonukleotid der Formel V gemäss Anspruch 33, dadurch gekennzeichnet, dass y für eine Zahl von 0 bis 100 steht.

**37.** Oligonukleotid der Formel V gemäss Anspruch 36, dadurch gekennzeichnet, dass y für eine Zahl von 0 bis 50 steht.

**38.** Oligonukleotid der Formel V gemäss Anspruch 37, dadurch gekennzeichnet, dass y für eine Zahl von 0 bis 20 steht.

**39.** Oligonukleotid der Formel V gemäss Anspruch 33, dadurch gekennzeichnet, dass die Reste der Formeln VI und/oder VIa endständig und/oder in der Nukleotidsequenz gebunden sind.

**40.** Oligonukleotid der Formel V gemäss Anspruch 33, dadurch gekennzeichnet, dass die Reste der Formeln VI und/oder VIa zwischen Resten von natürlichen oder synthetischen Nukleosiden gebunden sind.

**41.** Oligonukleotid der Formel V gemäss den Ansprüchen 39 und 40, dadurch gekennzeichnet, dass 2 bis 5 gleiche oder verschiedene Reste der Formeln VI und/oder VIa aufeinander folgen.

**42.** Oligonukleotid der Formel V gemäss Anspruch 33, dadurch gekennzeichnet, dass insgesamt 4 bis 30 Nukleosideinheiten und 1 bis 12 Reste der Formeln VI und/oder VIa enthalten sind.

**43.** Oligonukleotid der Formel V gemäss Anspruch 33, worin B als Purinrest oder einem Analogen davon einen Rest der Formeln II, IIa, IIb, IIc, IId oder IIe darstellt,

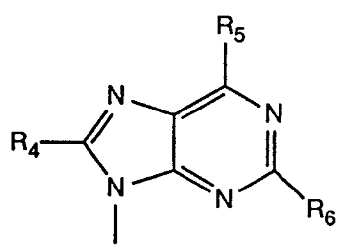

(II),

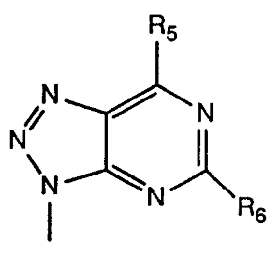

(IIa),

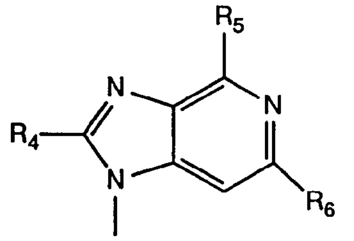

(IIb),

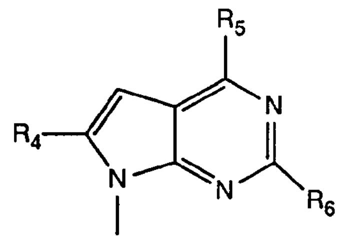

(IIc),

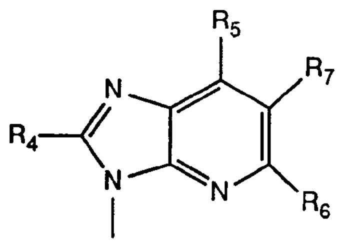

(IId),

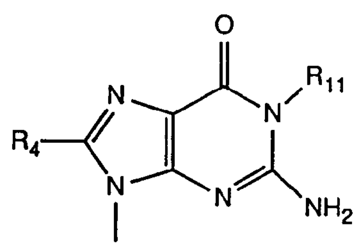

(IIe),

worin $R_4$ für H, Cl, Br oder OH oder $O(C_1-C_{12}$-Alkyl) steht, und $R_5$, $R_6$ und $R_7$ unabhängig voneinander H, OH, SH, $NH_2$, $NHNH_2$, NHOH, NHOAlkyl mit 1 bis 12 C-Atomen, $-N=CH-N(C_1-C_{12}$-Alkyl$)_2$, F, Cl, Br, Alkyl oder Hydroxyalkyl oder Aminoalkyl oder Alkoxy oder Alkylthio mit 1 bis 12 C-Atomen, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten, und $R_{11}$ H oder $C_1$-$C_4$-Alkyl darstellt.

**44.** Oligonukleotid der Formel V gemäss Anspruch 43, worin die Schutzgruppe für Hydroxyl- und Aminogruppen $C_1$-$C_8$-Acyl darstellt.

**45.** Oligonukleotid der Formel V gemäss Anspruch 43, worin das Primäramino 1 bis 12 C-Atome und das Sekundäramino 2 bis 12 C-Atome enthält.

**46.** Oligonukleotid der Formel V gemäss Anspruch 43, worin es sich bei dem Primäramino und Sekundäramino um Reste der Formel $R_5R_9N$ handelt, worin $R_8$ für H steht oder unabhängig die Bedeutung von $R_9$ hat, und $R_9$ $C_1$-$C_{20}$-Alkyl, -Aminoalkyl, -Hydroxyalkyl; Carboxyalkyl oder Carbalkoxyalkyl, wobei die Carbalkoxygruppe 2 bis 8 C-Atome enthält und die Alkylgruppe 1 bis 6, bevorzugt 1 bis 4 C-Atome; $C_2$-$C_{20}$-Alkenyl; Phenyl, Mono- oder Di-($C_1$-$C_4$-Alkyl- oder Alkoxy)phenyl, Benzyl, Mono- oder Di-($C_1$-$C_4$-

Alkyl- oder -Alkoxy)benzyl; oder 1,2-, 1,3- oder 1,4-Imidazolyl-$C_1$-$C_6$-Alkyl darstellt, oder $R_8$ und $R_9$ zusammen Tetra- oder Pentamethylen, 3-Oxa-1,5-pentylen, $-CH_2-NR_{10}-CH_2CH_2-$ oder $-CH_2CH_2-NR_{10}-CH_2CH_2-$ darstellen, worin $R_{10}$ für H oder $C_1$-$C_4$-Alkyl steht, wobei die Aminogruppe im Aminoalkyl unsubstituiert oder mit ein oder zwei $C_1$-$C_4$-Alkyl oder -Hydroxyalkylgruppen substituiert ist, und die Hydroxylgruppe im Hydroxyalkyl gegebenenfalls mit $C_1$-$C_4$-Alkyl verethert ist.

**47.** Oligonukleotid der Formel V gemäss Anspruch 46, worin es sich bei dem Primäramino und Sekundäramino um Methyl-, Ethyl-, Dimethyl-, Diethyl-, Allyl-, Mono- oder Di-(hydroxy-eth-2-yl)-, Phenyl- und Benzyl-, Acetyl- oder Benzoylamino handelt.

**48.** Oligonukleotid der Formel V gemäss Anspruch 43, worin $R_4$ in den Formeln II, IIb, IIc, IId und IIe für Wasserstoff steht.

**49.** Oligonukleotid der Formel V gemäss Anspruch 43, worin $R_7$ in Formel IId für Wasserstoff steht.

**50.** Oligonukleotid der Formel V gemäss Anspruch 43, worin $R_5$ und $R_6$ in den Formeln II, IIa, IIb, IIc, IId und IIe unabhängig voneinander H, F, Cl, Br, OH, SH, $NH_2$, NHOH, $NHNH_2$, Methylamino, Dimethylamino, Benzoylamino, Methoxy, Ethoxy oder Methylthio darstellen.

**51.** Oligonukleotid der Formel V gemäss Anspruch 43, worin B ein Purinrest oder ein Rest eines Purinanalogen aus der Reihe Adenin, N-Methyladenin, 2-Methylthioadenin, 2-Aminoadenin, 6-Hydroxypurin, 2-Amino-6-chlorpurin, 2-Amino-6-methylthiopurin, Guanin und N-Isobutyrylguanin ist.

**52.** Oligonukleotid der Formel V gemäss Anspruch 33, worin B als analoger Pyrimidinrest einen Uracil-, Thymin- oder Cytosinrest der Formeln III, IIIa und IIIb darstellt,

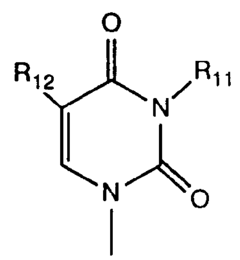

(III),

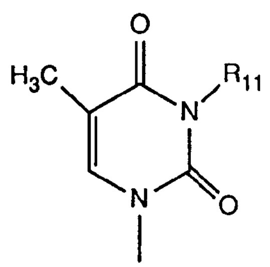

(IIIa)

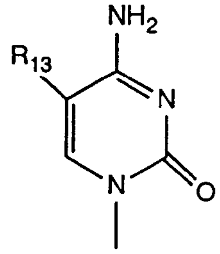

(IIIb),

worin $R_{11}$ H oder $C_1$-$C_4$-Alkyl bedeutet, und $R_{12}$ und $R_{13}$ unabhängig voneinander H, OH, SH, $NH_2$, $NHNH_2$,

NHOH, NHOAlkyl mit 1 bis 12 C-Atomen, F, Cl, Br, Alkyl oder Hydroxyalkyl oder Aminoalkyl oder Alkoxy oder Alkylthio mit 1 bis 12 C-Atomen, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten, und die Wasserstoffatome der $NH_2$-Gruppe in Formel IIIb unsubstituiert oder mit $C_1$-$C_6$-Alkyl, Benzoyl oder Benzyl substituiert sind, sowie die Dihydroderivate der Reste der Formeln III, IIIa und IIIb.

**53.** Oligonukleotid der Formel V gemäss Anspruch 52, worin $R_{12}$ H, $C_1$-$C_6$-Alkyl oder -Hydroxyalkyl, F, Cl, Br, $NH_2$, Benzoylamino, Mono- oder Di-$C_1$-$C_6$-alkylamino darstellt.

**54.** Oligonukleotid der Formel V gemäss Anspruch 52, worin $R_{13}$ H, $C_1$-$C_6$-Alkyl oder -Alkoxy oder -Hydroxyalkyl, F, Cl, Br, $NH_2$, Benzoylamino, Mono- oder Di-$C_1$-$C_6$-alkylamino darstellt.

**55.** Oligonukleotid der Formel V gemäss Anspruch 53, worin $R_{12}$ H, F, Cl, Br, $NH_2$, $NHCH_3$, $N(CH_3)_2$ oder $C_1$-$C_4$-Alkyl bedeutet.

**56.** Oligonukleotid der Formel V gemäss Anspruch 55, worin $R_{13}$ H, $C_1$-$C_4$-Alkyl, $NH_2$, $NHCH_3$ oder $(CH_3)_2N$ bedeutet.

**57.** Oligonukleotid der Formel V gemäss Anspruch 52, worin B als Rest eines Pyrimidinanalogen sich von Uracil, Thymin, Cytosin, 5-Fluoruracil, 5-Cloruracil, 5-Bromuracil, Dihydrouracil oder 5-Methylcytosin ableitet.

**58.** Oligonukleotid der Formel V gemäss Anspruch 33, worin y eine Zahl von 2 bis 50 darstellt, Y für die Gruppe $-P(O)O^{\ominus}-$ steht, U, V und W je für sich gleiche oder verschiedene Reste eines natürlichen oder synthetischen Nukleosids bedeuten und mindestens einer der Reste U, V oder W der Formel VI oder VIa entspricht, A für $-CH_2-$ steht und B der Rest einer natürlichen Nukleosidbase ist.

**59.** Oligonukleotid der Formel V gemäss Anspruch 58, worin es sich bei den Resten eines natürlichen Nukleosids um solche von Adenosin, Cytidin, Guanosin, Uridin, Thymidin, 2'-Desoxyadenin, 2'-Desoxycytidin und 2'-Desoxyguanosin handelt.

**60.** Oligonukleotid der Formel V gemäss Anspruch 58, worin es sich bei den Resten B als natürliche Nukleosidbasen um Adenin, Cytosin, Guanin, Thymin, Uracil, 2-Aminoadenin oder 5-Methylcytosin handelt.

**61.** Oligonukleotid der Formel V gemäss Anspruch 58, worin die Reste der Formeln VI und/oder VIa endständig und/oder in der Nukleotidsequenz gebunden sind.

**62.** Oligonukleotid der Formel V gemäss Anspruch 58, dadurch gekennzeichnet, dass die Reste der Formeln VI und/oder VIa zwischen Resten von natürlichen oder synthetischen Nukleosiden gebunden sind.

**63.** Oligonukleotid der Formel V gemäss den Ansprüchen 61 und 62, dadurch gekennzeichnet, dass 2 bis 5 gleiche oder verschiedene Reste der Formeln VI und/oder VIa aufeinander folgen.

**64.** Oligonukleotid der Formel V gemäss Anspruch 58, dadurch gekennzeichnet, dass y eine Zahl von 2 bis 20 darstellt und 1 bis 12 Reste der Formeln VI und/oder VIa enthalten sind.

**65.** Verwendung eines Oligonukleotids der Formel V als Diagnostikum zum Nachweis von viralen Infektionen oder genetisch bedingten Krankheiten.

**66.** Oligonukleotid der Formel V zur Anwendung in einem therapeutischen Verfahren zur Behandlung von Krankheiten bei Warmblütern einschliesslich des Menschen durch Wechselwirkung mit Nukleotidsequenzen im Körper.

**67.** Pharmazeutisches Präparat, enthaltend eine wirksame Menge eines Nukleosids der Formeln I und/oder Ia oder eines Oligonukleotids der Formel V alleine oder zusammen mit anderen Wirkstoffen, ein pharmazeutisches Trägermaterial und gegebenenfalls Hilfsstoffe.